(19) Europäisches Patentamt
European Patent Office
Office européen des brevets

(11) **EP 4 083 067 A1**

(12) **EUROPEAN PATENT APPLICATION**
published in accordance with Art. 153(4) EPC

(43) Date of publication:
02.11.2022 Bulletin 2022/44

(21) Application number: 20889776.9

(22) Date of filing: 23.11.2020

(51) International Patent Classification (IPC):
*C07K 16/18* (2006.01)   *A61K 38/05* (2006.01)
*A61P 37/02* (2006.01)   *C12N 5/10* (2006.01)
*C12N 15/62* (2006.01)

(86) International application number:
PCT/CN2020/130945

(87) International publication number:
WO 2021/098882 (27.05.2021 Gazette 2021/21)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 21.11.2019 CN 201911150678

(71) Applicant: Persongen Biotherapeutics (Suzhou)
Co., Ltd.
Suzhou, Jiangsu 215000 (CN)

(72) Inventors:
• YANG, Lin
Suzhou, Jiangsu 215000 (CN)

• YOU, Fengtao
Suzhou, Jiangsu 215000 (CN)
• CHEN, Dan
Suzhou, Jiangsu 215000 (CN)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

Remarks:
The complete document including Reference
Table(s) and the Sequence Listing(s) can be
downloaded from the EPO website

(54) **CD7-CAR-T CELL AND PREPARATION AND APPLICATION THEREOF**

(57) A CD7-CAR-T cell and preparation and application thereof. Specifically, the present invention provides a chimeric antigen receptor (CAR), and an antigen binding domain of the CAR comprises one or more CD7 nanobodies. The present invention further provides a CAR-T cell containing the CAR, and an endogenous CD7 expression of the CAR-T cell is blocked. The CAR-T cell can be used for treatment of T cell tumors.

EP 4 083 067 A1

**Description**

**Technical Field**

The present invention is related to the field of biotechnology, and more particularly to a CD7-CAR-T cell and preparation and use thereof.

**Background**

T-cell derived malignancies represent a typical type of blood system cancer, with quite high recurrence and mortality rates in both pediatric and adult patients. There is currently no highly effective therapy. T-cell acute lymphoblastic leukemia (T-ALL) is a highly heterogeneous hematological malignancy, accounting for approximately 25% of adult acute lymphoblastic leukemia cases and approximately 15% of pediatric acute lymphoblastic leukemia cases. Clinical symptoms of T-ALL mainly include infection, anemia, fever, abnormal bleeding and the like. T-ALL progresses rapidly and can infiltrate into lymph nodes, liver, spleen, central nervous system and testicles and other tissues and organs in a short period. Patients may die soon after the onset of the disease in case of no promptly and effective treatment. Currently, treatment strategies of T-cell acute lymphoblastic leukemia (T-ALL) mainly include intensive chemotherapy, allogeneic hematopoietic stem cell transplantation (allo-HSCT), antiviral therapy and molecular targeted therapy. However, intensive chemotherapy usually can not prevent disease recurrences after treatment. For those patients who relapse after the first treatment, the remission rate of salvage chemotherapy is only about 20-40%. In addition, although hematopoietic stem cell transplantation may be the only curable option at present, it may also be associated with a life-threatening risk caused by graft-versus-host diseases.

In recent years, chimeric antigen receptor T cell (CAR-T) therapy has shown very significant clinical therapeutic effects as a new adoptive immunotherapy technique for various solid tumors and hematological tumors, among which the treatment effect of therapy for B-cell derived lymphocytic leukemia and lymphoma is most significant. CAR-T therapy genetically modifies T lymphocytes of patients, in order to target and eliminate malignant tumors in a major histocompatibility complex-independent manner. One of the key factors to the effective application of this technology is to select a suitable target to construct corresponding CAR molecules. The best target antigen should be expressed only in tumor cells, not in normal cells, or may on certain cells that have a clinical response plan after the normal cells are temporarily killed.

CD7 is highly expressed in most T-cell acute lymphoblastic leukemia cells and some T-cell acute myeloid leukemia cells. Many research groups have reported that CD7 is highly expressed in T-cell acute lymphoblastic leukemia cells, but not in a group of normal T lymphocytes. CD7 molecule plays the role of co-stimulatory signal during T cell activation via binding with its ligand K12/SECTM1. However, it seems that CD7 does not have a key impact on the development and function of T cells. It was proved in some studies that mouse T progenitor cells, in which CD7 molecules were destroyed, still produced normal T cell development and homeostasis, and basically no change of T cell effector function was caused. Therefore, CD7 molecule may be a very suitable therapeutic target for T-cell acute lymphoblastic leukemia (T-ALL). However, the targeted treatment of T-cell acute lymphoblastic leukemia with CD7-CAR-T cells still faces great problems. This is because the expression of CD7 antigen in normal effector T cells and T-cell tumors will lead to self killing of CD7-CAR-T cells, so that it is difficult to successfully prepare CD7-CAR-T cells *in vitro.* Although recent reports have shown that in mouse experiments, T-ALL cells can be eliminated by CD7-CAR-T allogeneic CD7-CAR-T cells in which CD7 and T cell receptor are both knocked out, it is difficult to ensure that 100% knockout rate can be achieved in practical application, and there may still be a risk of graft-versus-host disease (GVHD) in clinical application.

Therefore, it is necessary to develop a new preparation method of CD7-CAR-T in this field, which can be amplified normally *in vitro* without affecting the function of CAR-T.

**Summary of Invention**

It is an object of the present invention to provide CD7-CAR-T cell as well as preparation method and application thereof.

Specifically, the present invention comprises the following contents:

1. In the present invention, a CD7 vector and method that effectively blocks expression of CD7 molecules on the surface of T cells are constructed.

2. In the present invention, 6 kinds of CD7-CAR-T cells are constructed based on anti-CD7 nanobody sequences and humanized antibody sequences.

3. In the present invention, it provides a structure and sequence of CD7-blocking plasmid.

4. In the present invention, it provides structures and sequences of 6 CD7-CAR vectors.

5. In the present invention, it provides the *in vivo* and *in vitro* killing results of CD7-CAR-T cells with different structures on T cell tumor cells.

6. In the present invention, it provides the *in vitro* killing results of CD7-CAR-T cells on CD7-positive acute myeloid leukemia cells.

In the first aspect of the present invention, it provides a chimeric antigen receptor (CAR), wherein an antigen binding domain of the CAR comprises one or more anti-CD7 nanobodies.

In another preferred embodiment, the amino sequence of the anti-CD7 nanobody is shown in SEQ ID NO: 24.

In another preferred embodiment, the CAR further comprises a transmembrane region derived from ICOS and/or an intracellular co-stimulatory region derived from ICOS.

In another preferred embodiment, the CAR comprises an intracellular co-stimulatory region derived from ICOS and an intracellular co-stimulatory region derived from 4-1BB.

In another preferred embodiment, the CAR comprises an intracellular co-stimulatory region derived from CD28 and an intracellular co-stimulatory region derived from 4-1BB.

In another preferred embodiment, the structure of the CAR is shown in the following Formula I:

$$\text{L-VHH-H-TM-C-CD3}\zeta \qquad \text{(I)}$$

wherein,

each "-" is independently a linking peptide or peptide bond;
L is a signal peptide sequence;
VHH is an antigen binding domain comprising one or two anti-CD7 nanobodies;
H is a hinge region;
TM is a transmembrane domain;
C is a co-stimulatory signaling molecule;
CD3$\zeta$ is a cytoplasmic signal transduction sequence derived from CD3$\zeta$.

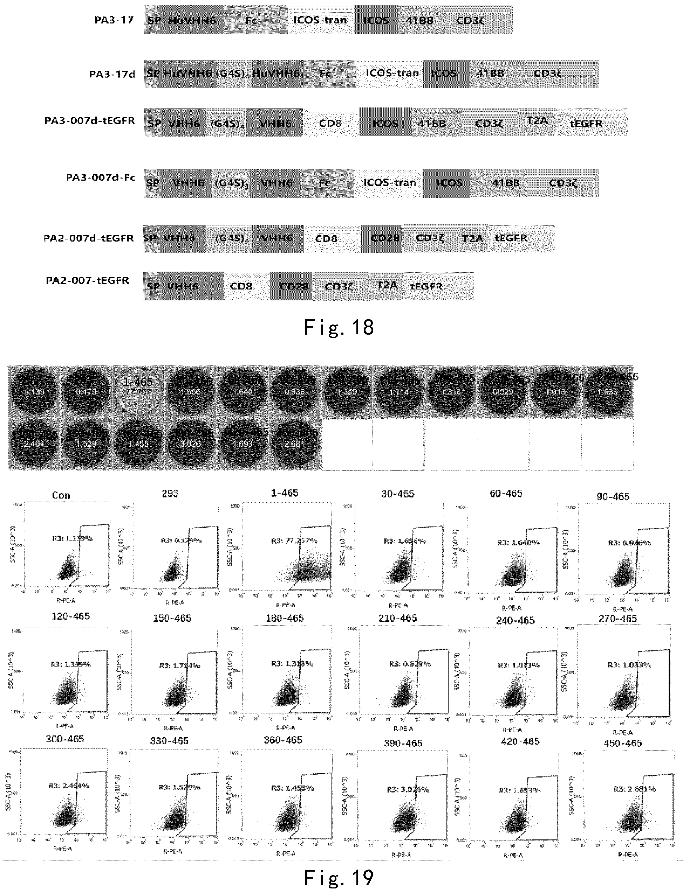

In another preferred embodiment, the structure of the CAR is shown in Fig. 18.

In another preferred embodiment, L is a signal peptide of a protein selected from the group consisting of CD8, CD28, GM-CSF, CD4, CD137 and a combination thereof.

In another preferred embodiment, L is a signal peptide derived from CD8.

In another preferred embodiment, the amino acid sequence of L is shown in SEQ ID NO: 4.

In another preferred embodiment, the VHH comprises two anti-CD7 nanobodies, preferably the two anti-CD7 nanobodies are connected with a linking peptide, and more preferably the linking peptide is shown in SEQ ID NO: 8.
in another preferred embodiment, the anti-CD7 nanobody comprises a humanized nanobody and a camel nanobody, and preferably a humanized nanobody.

In another preferred embodiment, the VHH is a humanized anti-CD7 nanobody.

In another preferred embodiment, H is a hinge region derived from a protein selected from the group consisting of Fc, CD8, CD28, CD137 and a combination thereof.

In another preferred embodiment, H is an Fc fragment, and preferably the Fc fragment is a human IgG4 Fc fragment.

In another preferred embodiment, the amino acid sequence of the Fc fragment is shown in SEQ ID NO: 25.

In another preferred embodiment, TM is a transmembrane region of a protein selected from the group consisting of ICOS, CD28, CD3 epsilon, CD45, CD4, CD5, CD8, CD9, CD16, CD22, CD33, CD37, CD64, CD80, CD86, CD134, CD137, CD154 and a combination thereof.

In another preferred embodiment, TM is a transmembrane region derived from ICOS.

In another preferred embodiment, the amino acid sequence of the transmembrane region derived from ICOS is shown in SEQ ID NO: 28.

In another preferred embodiment, C is a co-stimulatory signaling molecule of a protein selected from the group consisting of ICOS, OX40, CD2, CD7, CD27, CD28, CD30, CD40, CD70, CD 134, 4-1BB (CD137), PD1, Dap10, CDS, ICAM-1, LFA-1 (CD11a/CD18), ICOS (CD278), NKG2D, GITR, TLR2 and a combination thereof.

In another preferred embodiment, the C is a co-stimulatory signaling molecule derived from ICOS and/or 4-1BB.

In another preferred embodiment, the amino acid sequence of the co-stimulatory signaling molecule derived from ICOS is shown in SEQ ID NO: 29.

In another preferred embodiment, the amino acid sequence of the co-stimulatory signaling molecule derived from 4-1BB is shown in SEQ ID NO: 30.

In another preferred embodiment, the amino acid sequence of CD3ζ is shown in SEQ ID NO: 31.

In another preferred embodiment, the CAR further comprises a cell suicide element (preferably at C-terminus).

In another preferred embodiment, the CAR is connected with the cell suicide element via a self-cleaving element.

In another preferred embodiment, the cell suicide element is connected with Cd3ζ of the CAR via T2A.

In another preferred embodiment, the cell suicide element is selected from the group consisting of HSV-TK, iCasp9, ΔCD20, mTMPK, ΔCD19, RQR8, EGFRt and a combination thereof.

In another preferred embodiment, the cell suicide element is tEGFR.

In another preferred embodiment, the amino acid sequence of the tEGFR is shown in SEQ ID NO: 23.

In another preferred embodiment, the amino acid sequence of T2A is shown in SEQ ID NO: 32.

In another preferred embodiment, the CAR is PA3-17 CAR.

In another preferred embodiment, the amino acid sequence of the CAR is shown in SEQ ID NO: 20.

In another preferred embodiment, the CAR recognizes an antigenic epitope shown in positions 28-37 of SEQ ID NO: 49.

In the second aspect of the present invention, it provides a CD7-blocking molecule, which comprises one or more anti-CD7 nanobodies and an endoplasmic reticulum retention sequence.

In another preferred embodiment, the CD7-blocking molecule has a structure as shown in the following Formula II:

$$L'\text{-}VHH'\text{-}ER \qquad (II)$$

wherein,

each "-" is independently a linking peptide or peptide bond;
L' is a signal peptide sequence;
VHH' is an binding region comprising two anti-CD7 nanobodies; and
ER is an endoplasmic reticulum retention sequence.

In another preferred embodiment, the amino acid sequence of L' is shown in SEQ ID NO: 4.

In another preferred embodiment, the two anti-CD7 nanobodies in the VHH' are connected with a linking peptide, and more preferably the linking peptide is shown in SEQ ID NO: 8.

In another preferred embodiment, the amino acid sequence of the VHH' is shown in SEQ ID NO: 6.

In another preferred embodiment, the VHH' is a camel nanobody.

In another preferred embodiment, the amino acid sequence of the ER is shown in SEQ ID NO: 10.

In another preferred embodiment, the amino acid sequence of the CD7-blocking molecule is shown in SEQ ID NO: 2.

In another preferred embodiment, the nucleotide sequence of the CD7-blocking molecule is shown in SEQ ID NO: 1.

In the third aspect of the present invention, it provides a nucleic acid molecule encoding the CAR of the first aspect and/or the CD7-blocking molecule of the second aspect of the present invention.

In the fourth aspect of the present invention, it provides a vector comprising the nucleic acid molecule of the third aspect of the present invention.

In another preferred embodiment, the vector is selected from the group consisting of DNA, RNA, plasmid, lentiviral vector, adenoviral vector, retroviral vector, transposon and a combination thereof.

In another preferred embodiment, the vector is a lentiviral vector.

In the fifth aspect of the present invention, it provides a host cell comprising the vector of the fourth aspect of the present invention, or having the exogenous nucleic acid molecule of the third aspect of the present invention integrated into the chromosome, or expressing the CAR of the first aspect and/or the CD7-blocking molecule of the second aspect of the present invention.

In the sixth aspect of the present invention, it provides an engineered immune cell comprising the vector of the fourth aspect of the present invention, or having the exogenous nucleic acid molecule of the third aspect of the present invention integrated into the chromosome, or expressing the CAR of the first aspect and/or the CD7-blocking molecule of the second aspect of the present invention.

In another preferred embodiment, gene expression of CD7 on surface of the immune cell is silenced.

In another preferred embodiment, the immune cell is a T cell, whose TCR gene is silenced.

In another preferred embodiment, the immune cell expresses exogenous cell suicide element.

In another preferred embodiment, the engineered immune cell is selected from the following group:

(i) chimeric antigen receptor T cells (CAR-T cells); or
(ii) chimeric antigen receptor NK cells (CAR-NK cells).

In another preferred embodiment, in the immune cell, the CAR and the cell suicide element are co-expressed.
In another preferred embodiment, the immune cell is a T cell, and the T cell is a CD4+ T cell, and preferably the T cell secretes IL-6.
In the eighth aspect of the present invention, it provides a formulation which comprises the engineered immune cell of the sixth aspect of the present invention, and a pharmaceutically acceptable carrier, diluent or excipient.
In another preferred embodiment, the formulation is a liquid preparation.
In another preferred embodiment, the dosage form of the formulation is injection.
In another preferred embodiment, the concentration of the engineered immune cell in the formulation is $1\times10^3$-$1\times10^8$ cells/ml, and preferably $1\times10^4$-$1\times10^7$ cells/ml.
In the ninth aspect of the present invention, it provides the use of the CAR of the first aspect of the present invention, the CD7-blocking molecule of the second aspect, or the engineered immune cell of the sixth aspect of the present invention, in preparation of a medicine or a formulation for preventing and/or treating tumor or cancer.
In another preferred embodiment, the tumor is a T cell tumor.
In another preferred embodiment, the tumor is a hematological tumor.
In another preferred embodiment, the hematological tumor is selected from the group consisting of acute myeloid leukemia (AML), multiple myeloma (MM), chronic lymphocytic leukemia (CLL), acute lymphoblastic leukemia (ALL), diffuse large B cell lymphoma (DLBCL) and a combination thereof.
In another preferred embodiment, the medicine or formulation is used for preventing and/or treating acute myeloid leukemia (AML).
In the tenth aspect of the present invention, it provides a method for preparing an engineered immune cell expressing the CAR of the first aspect and/or the CD7-blocking molecule of the second aspect of the present invention, which comprises a step of:
transducing the nucleic acid molecule of the third aspect of the present invention or the vector of the fourth aspect of the present invention into an immune cell, thereby obtaining the engineered immune cell.
In another preferred embodiment, the immune cell is a T cell or a NK cell.
In the eleventh aspect of the present invention, it provides a method for preparing an engineered immune cell, which comprises the steps of:

(1) providing an immune cell to be modified;
(2) transducing a first expression cassette expressing the CAR of the first aspect of a present invention into the immune cell; and
(3) transducing a second expression cassette expressing the CD7-blocking molecule of a second aspect of the present invention into the immune cell, thereby obtaining the engineered immune cell.

In another preferred embodiment, step (2) is performed before, after, at the same time, or alternately with step (3), and preferably step (2) and step (3) are performed at the same time.
In another preferred embodiment, in step (2), a first virus vector comprising the first expression cassette expressing the CAR of the first aspect of the present invention is transduced into the immune cell.
In another preferred embodiment, in step (3), a second virus vector comprising the second expression cassette expressing the CD7-blocking molecule of the second aspect of the present invention is transduced into the immune cell.
In another preferred embodiment, in step (3), the second expression cassette introduced into the immune cell can block endogenous CD7 expression of the immune cell, by at least 20%, preferably 10%, more preferably 5%, and most preferably 2.5%.
In the twelfth aspect of the present invention, it provides a kit for preparation of the engineered immune cell of the sixth aspect of the present invention, wherein the kit comprises a container, in which the nucleic acid molecule of the third aspect of the present invention or the vector of the fourth aspect of the present invention is located.
In the thirteenth aspect of the present invention, it provides a kit for preparation of the engineered immune cell of the sixth aspect of the present invention, wherein the kit comprises a container and, located within the container:

(1) a first virus vector, which comprises a first expression cassette expressing the CAR of the first aspect of the present invention; and,
(2) a second virus vector, which comprises a second expression cassette expressing the CD7-blocking molecule of the second aspect of the present invention.

In another preferred embodiment, the first and second cassettes are located in the same or different containers.

In another preferred embodiment, the first and second cassettes are located in the same expression vector.

In the fourteenth aspect of the present invention, it provides an reagent combination comprising:

(1) a first virus vector, which comprises a first expression cassette expressing the CAR of the first aspect of the present invention; and,

(2) a second virus vector, which comprises a second expression cassette expressing the CD7-blocking molecule of the second aspect of the present invention.

In another preferred embodiment, the first and second virus vectors are lentiviral vectors.

In another preferred embodiment, the first and second expression cassettes are lentiviral vectors of the same type.

In another preferred embodiment, the reagent combination is used for preparation of the engineered immune cell of the sixth aspect of the present invention.

In the fifteenth aspect of the present invention, it provides a use of the engineered immune cell of the sixth aspect of the present invention, for the prevention and/or treatment of cancer or tumor.

In another preferred embodiment, the cancer or tumor is a T cell tumor.

In the sixteenth aspect of the present invention, it provides a method of treating a disease which comprises: administering an appropriate amount of the cell of the sixth aspect of the present invention, or the formulation of the eighth aspect of the present invention, to a subject in need of treatment.

In another preferred embodiment, the disease is cancer or tumor.

In another preferred embodiment, the cancer or tumor is a hematological tumor, preferably an acute myeloid leukemia (AML)

It should be understood that within the scope of the present invention, each technical features of the present invention described above and in the following (as examples) may be combined with each other to form a new or preferred technical solution, which is not redundantly described one-by-one herein due to space limitation.

**Description of Figure**

Figure 1 shows the effect of blocking CD7 on CD7 expression on the surface of T cells. Figure 1A shows the positive rate of CD7 on the surface of control T cells. Figure 1B shows the positive rate of CD7 on the surface of T cells after blocking CD7.

Figure 2 shows the effect of blocking CD7 on the expansion of T cells. Ctrl T represents T cells, CD7-CAR represents T cells transfected with CD7-CAR virus only, and CD7-blocking & CD7-CAR represents T cells transfected with both CD7-CAR virus and CD7-blocking virus.

Figure 3 shows the effect of blocking CD7 on T cell phenotype. Blank represents control T cells, CD19-CAR represents CD19-CAR-T cells, CD7-blocking & CD19-CAR represents CD7-blocked CD19-CAR-T cells, CD7-blocking represents CD7-blocked T cells, and CD7-blocking & CD7-CAR represents CD7-blocked CD7-CAR-T cells. Figure 3A shows the results of flow cytometry on Day 7. Figure 3B shows the results of flow cytometry on Day 14.

Figure 4 shows the effect of blocking CD7 on CAR-T cell function. Figure 4A shows the expression of CD7 and CD19-CAR on the surface of CD7-blocked CD19-CAR-T cells (CD7-blocking & CD19-CAR) and control CD19-CAR-T cells (CD19-CAR). Figure 4B shows the cytotoxicity of two kinds of CAR-T cells on Raji cells under the effector:target ratio of 1:1. Figure 4C shows the cytotoxicity of two kinds of CAR-T cells on Raji cells under the effector:target ratio of 5:1.

Figure 5 shows the effect of blocking CD7 on cytokine secretion of CAR-T cells. CD7-blocking represents CD7-blocked T cells, and CD7-blocking & CD19-CAR represents CD7-blocked CD19-CAR-T cells.

Figure 6 shows the changes of CD69 phenotype after CD7-CAR-Jurkat cells were incubated with target cells.

Figure 7 shows the transfection efficiency of CD7-CAR-T with different structures.

Figure 8 shows the killing results of CD7-CAR-T cells on CD7-positive tumor cells *in vitro*. Figure 8A shows the killing results of CD7-CAR-T cells on CEM cells. Figure 8B shows the killing results of CD7-CAR-T cells on Jurkat cells.

Figure 9 shows the release of cytokines after CD7-CAR-T cells were incubated with target cells.

Figure 10 shows the killing results of CD7-CAR-T cells on CD7-overexpressing cells 293-T cells.

Figure 11 shows the release of cytokines after CD7-CAR-T cells were incubated with 293T-CD7 cells.

Figure 12 shows the cytotoxicity of CD7-CAR-T cells on primary tumor cells.

Figure 13 shows the preparation and flow cytometry of CD4-positive CD7-CAR-T cells and CD8-positive CD7-CAR-T cells. Figure 13A shows the flow cytometry results of sorted CD4-positive T cells and CD8-positive T cells. Figure 13b shows the flow cytometry results of CD7 and CD7-CAR on the surface of two kinds of CAR-T cells.

Figure 14 shows the sustained *in vitro* results of CD4+ CD7-CAR-T cells and CD8+ CD7-CAR-T cells on CEM cells. Figure 14A shows a schematic diagram of CAR-T cells multi-stimulated by antigens. Figure 14B shows the results of repeated killing of CEM by CD4+ CD7-CAR-T cells and CD8+ CD7-CAR-T cells.

Figure 15 shows the release of cytokines from CD4+ CD7-CAR-T cells and CD8+ CD7-CAR-T cells multi-stimulated by antigens.

Figure 16 shows the animal experimental scheme and the survival curve of mice.

Figure 16A shows the animal experiment scheme. Figure 16b shows the survival curve of current mice.

Figure 17 shows the structure of CD7-blocking sequence.

Figure 18 shows the structural diagram of different CD7-CAR vectors.

Figure 19 shows the results of flow cytometry detection of anti-CD7 antibodies binding to different truncated antigens. Among them, the above panel shows the results of ELISA and the below panel shows the results of flow cytometry.

Figure 20 shows the results of flow cytometry detection of the anti-CD7 antibodies binding to antigens with mutations at different sites.

Figure 21 shows the results of the membrane proteome array experiment of anti-CD7 antibodies.

Figure 22 shows the detection of expression rate of CD7 antigen on the surfaces of six AML tumor cell lines. Among them, Figures 22A-22F show the detection of expression rate of CD7 antigen on the surfaces of KG-1 cells, HEL cells, THP-1 cells, HL60 cells, AML5 cells and Kasumi-1 cells, respectively.

Figure 23 shows the construction and preparation of CD7-CAR T cells. Among them, Figure 23A shows the structures of CD7-CAR and PEBL, Figure 23B shows the blocking effect of PEBL, Figure 23C shows the virus titer of CD7-CAR, and Figure 23d shows the expression rate of CD7-CAR.

Figure 24 shows that CD7-CAR T has specific killing effect on 293T cells expressing CD7. Among them, Figure 24A shows the growth curves of 293T cells and 293T-CD7 cells after adding T cells and CD7-CAR T cells respectively, and Figure 24B shows the secretion of growth factors.

Figure 25 shows that CD7-CAR T has a strong killing effect on AML cells expressing CD7. Among them, Figure 25A shows the apoptosis of AML tumor cells after incubation of effector cells and target cells at the ratio of 1:1 for 48 hours, and Figure 25B shows the secretion of growth factors.

## EMBODIMENTS

After extensive and intensive research, the inventors have constructed a CD7-blocking vector that is able to block the CD7 expression on the surface of T cell and a CD7-CAR vector comprising a anti-CD7 nanobody. The inventors further constructed a CD7-CAR-T cell, in which CD7 expression on the surface is blocked. It is indicated in experiments that the CD7-CAR-T cell of the present invention can be normally amplified *in vitro* and can specifically kill CD7 positive malignant tumors. On this basis, the present invention has been completed.

Specifically, firstly, in the present invention, a CD7-blocking vector that can block the CD7 expression on the surfaces of T cells was constructed by using anti-CD7 nanobody sequence. It has been proven in experiments that the CD7-blocking vector constructed in the present invention can effectively block the normal CD7 molecule expression on the surface of T cell.

Secondly, in the present invention, six CD7-CAR vectors with different structures were constructed by using anti-CD7 nanobody sequence. In the present invention, it discloses the relevant fragments and specific sequences of the 6 kinds of CD7-CAR vectors, including sequences of anti-CD7 nanobody, humanized anti-CD7 nanobody, and hinge region, transmembrane region and co-stimulatory signal in different CAR structures. The CD7-CAR of the present invention comprises a third-generation CAR comprising an ICOS and 41B co-stimulatory signal, and a second-generation CAR comprising only a CD28 co-stimulatory signal.

Based on the above CD7-blocking vector and CD7-CAR vector, in the present invention, a series of CD7-CAR-T cells are constructed, which can be normally amplified *in vitro* and will not cause self-killing of CAR-T cells, while having significant specific killing effects on CD7-positive T cell malignant tumors, thereby establishing a foundation for clinically targeted therapy of T cell-related malignant tumors (T-cell leukemia and lymphoma). Further, the CD7-CAR-T cell constructed in the present invention also exhibits superior killing effects *in vitro* on T cell tumor cells CEM and primary T-ALL tumor cells derived from tumor patients.

## Terms

To make the disclosure easier to understand, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the present invention into a subject using any one of various methods and delivery systems known to those skilled in the art, including intravenous, intramuscular, subcutaneous, intraperitoneal, spinal or other parenteral administration, such as by injection or infusion.

The term "antibody" (Ab) may include, but is not limited to, an immunoglobulin that specifically binds an antigen and contains at least two heavy (H) chains and two light (L) chains linked by disulfide bonds, or an antigen binding parts thereof. Each H chain contains a heavy chain variable region (abbreviated herein as VH) and a heavy chain constant region. The heavy chain constant region contains three constant domains, CH1, CH2, and CH3. Each light chain contains a light chain variable region (abbreviated herein as VL) and a light chain constant region. The light chain constant region contains a constant domain CL. The VH and VL regions can be further subdivided into hypervariable regions called complementarity determining regions (CDR), which are interspersed within more conservative regions called framework regions (FR). Each VH and VL contains three CDRs and four FRs, which are arranged from amino terminal to carboxy terminal in the following order: FR1, CDR1, FR2, CDR2, FR3, CDR3, FR4. The variable regions of the heavy and light chains contain a binding domain that interacts with an antigen.

It should be understood that the names of amino acids herein are represented by the internationally accepted single English letter, to which the corresponding three English letter names of amino acid are as follows: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y) and Val (V).

The meanings represented by each sequence provided in the sequence list of this application are shown in following Table. 1.

Table. 1 Names of sequences in the sequence list

| SEQUENCE | NAME |
| --- | --- |
| SEQ ID NO: 1 | Base sequence of CD7-blocking molecule |
| SEQ ID NO: 2 | Amino acid sequence of CD7-blocking molecule |
| SEQ ID NO: 3 | Base sequence of sp (signal peptide) |
| SEQ ID NO: 4 | Amino acid sequence of sp (signal peptide) |
| SEQ ID NO: 5 | Base sequence of anti-CD7 nanobody (VHH6) |
| SEQ ID NO: 6 | Amino acid sequence of anti-CD7 nanobody (VHH6) |
| SEQ ID NO: 7 | Base sequence of (G4S)4 |
| SEQ ID NO: 8 | Amino acid sequence of (G4S)4 |
| SEQ ID NO: 9 | Base sequence of ER (endoplasmic reticulum retention sequence) |
| SEQ ID NO: 10 | Amino acid sequence of ER (endoplasmic reticulum retention sequence) |
| SEQ ID NO: 11 | Base sequence of PA2-007-tEGFR |
| SEQ ID NO: 12 | Amino acid sequence of PA2-007-tEGFR |
| SEQ ID NO: 13 | Base sequence of PA2-007d-tEGFR |
| SEQ ID NO: 14 | Amino acid sequence of PA2-007d-tEGFR |
| SEQ ID NO: 15 | Base sequence of PA3-007d-tEGFR |
| SEQ ID NO: 16 | Amino acid sequence of PA3-007d-tEGFR |
| SEQ ID NO: 17 | Base sequence of PA3-007d-Fc |
| SEQ ID NO: 18 | Amino acid sequence of PA3-007d-Fc |
| SEQ ID NO: 19 | Base sequence of PA3-17 |
| SEQ ID NO: 20 | Amino acid sequence of PA3-17 |
| SEQ ID NO: 21 | Base sequence of PA3-17d |
| SEQ ID NO: 22 | Amino acid sequence of PA3-17d |
| SEQ ID NO: 23 | Amino acid sequence of tEGFR (truncated EGFR) |

(continued)

| SEQUENCE | NAME |
|---|---|
| SEQ ID NO: 24 | Amino acid sequence of HuVHH6 (humanized anti-CD7 nanobody sequence) |
| SEQ ID NO: 25 | Amino acid sequence of Fc (human IgG4 Fc sequence, hinge region) |
| SEQ ID NO: 26 | Amino acid sequence of CD8 (human CD8 hinge region sequence) |
| SEQ ID NO: 27 | Amino acid sequence of CD28 (CD28 transmembrane region and intracellular region) |
| SEQ ID NO: 28 | Amino acid sequence of ICOS-tran (ICOS transmembrane region sequence) |
| SEQ ID NO: 29 | Amino acid sequence of ICOS (ICOS intracellular region sequence) |
| SEQ ID NO: 30 | Amino acid sequence of 4-1BB (4-1BB intracellular region sequence) |
| SEQ ID NO: 31 | Amino acid sequence of CD3ζ |
| SEQ ID NO: 32 | Amino acid sequence of T2A |
| SEQ ID NO: 33 | Truncated fragment of CD7 (Lenti-CD7(EPB1901)-puro 1-465) |
| SEQ ID NO: 34 | Truncated fragment of CD7 (Lenti-CD7(EPB1901)-puro 30-465) |
| SEQ ID NO: 35 | Truncated fragment of CD7 (Lenti-CD7(EPB1901)-puro 60-465) |
| SEQ ID NO: 36 | Truncated fragment of CD7 (Lenti-CD7(EPB1901)-puro 90-465) |
| SEQ ID NO: 37 | Truncated fragment of CD7 (Lenti-CD7(EPB1901)-puro 120-465) |
| SEQ ID NO: 38 | Truncated fragment of CD7 (Lenti-CD7(EPB1901)-puro 150-465) |
| SEQ ID NO: 39 | Truncated fragment of CD7 (Lenti-CD7(EPB1901)-puro 180-465) |
| SEQ ID NO: 40 | Truncated fragment of CD7 (Lenti-CD7(EPB1901)-puro 210-465) |
| SEQ ID NO: 41 | Truncated fragment of CD7 (Lenti-CD7(EPB1901)-puro 240-465) |
| SEQ ID NO: 42 | Truncated fragment of CD7 (Lenti-CD7(EPB1901)-puro 270-465) |
| SEQ ID NO: 43 | Truncated fragment of CD7 (Lenti-CD7(EPB1901)-puro 300-465) |
| SEQ ID NO: 44 | Truncated fragment of CD7 (Lenti-CD7(EPB1901)-puro 330-465) |
| SEQ ID NO: 45 | Truncated fragment of CD7 (Lenti-CD7(EPB1901)-puro 360-465) |
| SEQ ID NO: 46 | Truncated fragment of CD7 (Lenti-CD7(EPB1901)-puro 390-465) |
| SEQ ID NO: 47 | Truncated fragment of CD7 (Lenti-CD7(EPB1901)-puro 420-465) |
| SEQ ID NO: 48 | Truncated fragment of CD7 (Lenti-CD7(EPB1901)-puro 450-465) |
| SEQ ID NO: 49 | Antigenic epitope |
| SEQ ID NO: 50 | Signal peptide |
| SEQ ID NO: 51 | CD7-targeted CAR (whose co-stimulatory molecules are CD28 and 4-1BB) |

**Chimeric antigen receptor (CAR)**

The design of CARs has gone through the following process. The first generation CAR has only one intracellular signal component, CD3ζ or FcγRI molecule. Because there is only one activation domain in the cell, it can only cause transient T cell proliferation and less cytokine secretion, and does not provide long-term T cell proliferation signals and sustained antitumor effects *in vivo.* Therefore, it has not achieved very good clinical efficacy. In the second generation CAR, based on the original structure, a co-stimulatory molecule such as CD28, 4-1BB, OX40 and ICOS is introduced. Compared with the first generation CAR, the function has been greatly improved, and the sustainability of CAR-T cells and the ability to kill tumor cells are further enhanced. Based on the second generation CAR, some new immune stimulatory molecules such as CD27 and CD134 are linked in tandem to develop the third and fourth generation CARs.

The chimeric antigen receptor (CAR) of the present invention comprises an extracellular domain, a transmembrane domain, and an intracellular domain. The extracellular domain comprises a target-specific binding element (also known as an antigen binding domain). The intracellular domain includes a co-stimulatory signaling region and a ζ chain.

The co-stimulatory signaling region refers to a part of the intracellular domain that includes a co-stimulatory molecule. The co-stimulatory molecule is a cell surface molecule required for efficient response of lymphocytes to antigens, rather than a antigen receptor or its ligand.

A linker (or linking peptide) can be incorporated between the extracellular domain and the transmembrane domain of the CAR, or between the cytoplasmic domain and the transmembrane domain of the CAR. As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids.

In a preferred embodiment of the present invention, the extracellular domain of the CAR provided by the present invention comprises an antigen binding domain targeting CD7. When the CAR of the present invention is expressed in T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to one or more intracellular domains of the co-stimulatory molecule and the $\zeta$ chain. Preferably, the antigen binding domain is fused with an intracellular domain of a combination of an ICOS and/or 4-1BB signaling domain and a CD3$\zeta$ signaling domain.

As used herein, the "antigen binding domain" refers to a Fab fragment, a Fab' fragment, a F (ab')$_2$ fragment, or a single Fv fragment that has antigen-binding activity. As a preferred embodiment of the present invention, the antigen binding domain contains one or more nanobodies which specifically recognize CD7.

As for the hinge region and the transmembrane region (transmembrane domain), the CAR can be designed to comprise a transmembrane domain fused to the extracellular domain of the CAR. In one embodiment, a transmembrane domain that is naturally associated with one of the domains in the CAR is used. In some embodiments, transmembrane domains may be selected or modified by amino acid substitutions to avoid binding such domains to the transmembrane domain of the same or different surface membrane proteins, thereby minimizing the interaction with other members of the receptor complexes.

The intracellular domain in the CAR of the present invention comprises the signaling domain of ICOS and/or 4-1BB and the signaling domain of CD3$\zeta$.

The present invention also provides CAR-T cells targeting CD7. Unless otherwise specified, the expression of CD7 on the surface of CAR-T cells of the invention is blocked, preferably by the CD7-blocking molecule described in the second aspect of the invention.

## Suicide gene switch

In order to further control the adverse effects of CAR-T cells such as non-tumor targeting and cytokine release syndrome, the CAR cells of the invention all contain suicide gene switches, which can effectively remove CAR-T cells in the body under the action of exogenous drugs, and block unknown or uncontrollable long-term toxicity, so as to ensure the safety of patients.

The suicide gene switch used in the invention may be HSV-TK, iCasp9, CD20, mTMPK, etc. In comparison, HSV TK, iCasp9 and CD20 have the same ability to remove T cells, but iCasp9 and CD20 remove faster and HSV-TK removes slower.

iCasp9 suicide gene contains a FKBP12-F36V domain, which can be connected via a flexible connector with cysteine aspartate protease 9, which does not contain recruitment domain. The FKBP12-F36V contains a FKBP domain in which phenylalanine replaces valine at the 36th amino acid residue. It has high selectivity and subnanomolar affinity, and can bind with synthetic dimerization ligands, such as AP1903 or other inert small molecules. When small molecules are added, dimerization is promoted and therefore cell apoptosis is induced, while there is ineffective for normal cells without suicide genes.

## Vector

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically.

The present invention also provides vectors in which the expression cassette of the present invention is inserted. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief, the expression cassette or nucleic acid sequence of the present invention is typically and operably linked to a promoter, and incorporated into an expression vector. The vectors can be suitable for replication and integration in eukaryotes. Typical cloning vectors contain transcription and translation terminators, initiation sequences, and promoters useful for regulation of the expression of the desired nucleic acid sequence.

The expression constructs of the present invention may also be used for nucleic acid immune and gene therapy, using standard gene delivery protocols. Methods for gene delivery are known in the art. See, e.g., U.S. Pat. Nos. 5,399,346, 5,580,859, and 5,589,466, which are incorporated by reference herein in entirety. In another embodiment, the present invention provides a gene therapy vector.

The nucleic acid can be cloned into various vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al, (2001 , Molecular Cloning: A Laboratory Manual, Cold Spring Harbor Laboratory, New York), and in other virology and molecular biology manuals. Viruses which are useful as vectors include but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for transferring a gene into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present invention should not be limited to the use of constitutive promoters. Inducible promoters are also contemplated as part of the present invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

In order to assess the expression of a CAR polypeptide or portions thereof, the expression vector to be introduced into a ceil can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the present invention, the vector is a lentiviral vector.

**Formulation**

The present invention provides a formulation (or preparation) comprising the CAR-T cell according to the first aspect of the present invention, and a pharmaceutically acceptable carrier, diluent or excipient. In one embodiment, the formulation is a liquid preparation. Preferably, the formulation is an injection. Preferably, the concentration of the CAR-T cells in the formulation is $1 \times 10^3$-$1 \times 10^8$ cells/ml, more preferably $1 \times 10^4$-$1 \times 10^7$ cells/ml.

In one embodiment, the formulation may comprises buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The formulation of the present invention is preferably formulated for intravenous administration.

**AML**

Acute myeloid leukemia (AML) is a group of highly heterogeneous and clonal hematopoietic stem cell diseases. Leukemia cases have been reported as early as 200 years ago. However, there has been little progress in the treatment of leukemia in the past few decades. The 5-year overall survival rate (OS) of young patients is about 40%, while for the vast majority of elderly patients with AML, the 5-year survival rate (OS) is only 10% - 20%. Moreover, there are still many defects, such as great harm to patients, high recurrence rate, difficulty to match, high cost and the like.

AML is a very intractable invasive hematopoietic stem cell malignant tumor as well as a disease that is very easy to relapse. Therefore, it is urgent to explore new treatment methods for relapsed and intractable AML. To develop new therapies for AML, attention should be paid not only to the efficiency, but also to the safety and toxicity to patients themselves.

Most of the current CAR-T treatments of AML have toxic and side effects on patients. Therefore, it is important to find an antigen that is selectively expressed only in malignant cells and lacking in normal cells. In the present application, it is demonstrated that CD7-CAR T cell can effectively target CD7-positive AML cells without cytotoxicity to cells that do not express CD7.

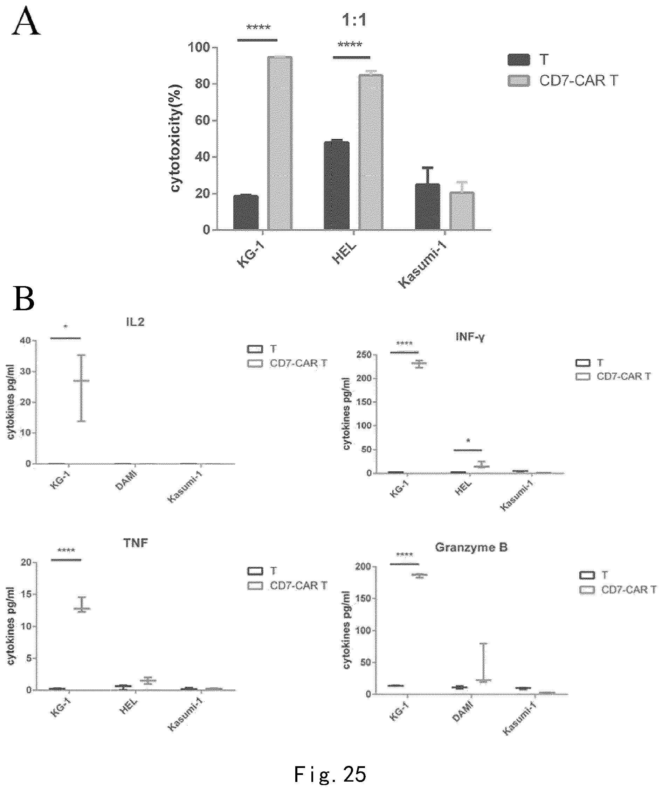

In the previous exploration experiment, the co-incubations were performed respectively with three effector:target ratios of 1:5, 1:1 and 5:1 for 24h, and with three effector:target ratios of 1:2, 1:1 and 2:1 for 24h and 48h. The detecting results were the same as in Fig.25. Therefore, it can be confirmed that CD7-CAR T indeed specifically kill CD7-positive tumor cells, and the killing effect is related to the level of CD7 antigen expression in tumor cells, and for tumor cells that do not express CD7 antigen, no specific killing activity was detected. Data of the *in vitro* experiments in the present application preliminarily proves that CD7-CAR T cells can efficiently kill CD7-positive tumor cells.

Although CD7-CAR T cells are not toxic to hematopoietic stem cells, they may have some toxic effects on normal CD7-positive T cells. However, fortunately, previous studies have shown that about 9% of normal T cells do not express CD7. These CD7-negative normal T cells have a sound T cell activity function. They can survive CD7-CAR-T cell therapy, thus providing patients with a basic immune protection and avoiding side effects similar to B cell hypoplasia caused by CD19-CAR-T cells. Therefore, the probability that CD7-CAR-T cell therapy results in T cell hypoplasia is quite low.

At present, a number of CAR-T products have been approved for marketing by FDA. CD19CAR, which was first tried to treat CD19-positive lymphoma, has achieved significant clinical efficacy. Moreover, CAR-T therapy has made great progress in both hematological tumors and solid tumors, and is considered to be one of the most promising tumor treatment methods. Although CD33 and CD123 are the most widely expressed targets for AML treatments today and have been shown to have some clinical efficacy potential, they cannot avoid severe hematopoietic toxic effects. Therefore, it is particularly urgent to develop new targets with good efficacy and safety. The present application provides a strong research basis for the clinical application of CD7 CAR-T, and brings new hope to patients with relapsed refractory AML.

**Therapeutic application**

The present invention comprises therapeutic applications by using cells (e.g., T cells) transduced with a lentiviral vector (LV) encoding the expression cassette of the present invention. The transduced T cells can target the tumor cell marker CD7, synergistically activate T cells, and cause T cell immune responses, thereby significantly increasing the killing efficiency against tumor cells.

Therefore, the present invention also provides a method for stimulating a T cell-mediated immune response to a target cell population or tissue in a mammal comprising a step of administering to the mammal a CAR-T cell of the present invention.

In one embodiment, the present invention comprises a cell therapy, wherein T cells from autologous patient (or heterologous donor) are isolated, activated and genetically modified to generate CAR-T cells, and then injected into the same patient. The probability of graft-versus-host-disease in the way is extremely low, and antigens are recognized by T cells in a non-MHC-restricted manner. In addition, one CAR-T can treat all cancers that express the antigen. Unlike antibody therapy, CAR-T cells are able to replicate *in vivo* and result in long-term persistence that can lead to sustained tumor control.

In one embodiment, the CAR-T cells of the present invention can undergo robust *in vivo* T cell expansion and can persist for an extended period. In addition, the CAR mediated immune response may be part of an adoptive immunotherapy approach in which CAR-modified T cells induce an immune response specific to the antigen binding moiety in the CAR. For example, an anti-CD7 CAR-T cell elicits an immune response specific against cells expressing CD7.

Although the data disclosed herein specifically disclose lentiviral vector comprising anti-CD7 nanobody, Fc fragment, transmembrane domain, and ICOS intracellular region, 4-1BB and CD3ζ signaling domains, it is contemplated that the present invention include any number of variations for each of the components of the construct as described elsewhere herein.

Cancers that may be treated include tumors that are unvascularized or largely unvascularized, and tumors that are vascularized. Cancers may include non-solid tumors (such as hematological tumors, for example, leukemias and lymphomas) or solid tumors. Types of cancers to be treated with the CARs of the present invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors (such as sarcomas and carcinomas) include fibrosarcoma, myxosarcoma, liposarcoma, mesothelioma, malignant lymphoma, pancreatic cancer and ovarian cancer.

The CAR-modified T cells of the present invention may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cells into a mammal: i) expanding the cells, ii) introducing a nucleic acid encoding a CAR to the cells, and/or iii) cryopreservation of the cells.

*Ex vivo* procedures are well known in the art and are discussed more fully below. Briefly, cells are isolated from a mammal (preferably a human) and genetically modified (*i.e.,* transduced or transfected *in vitro*) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of *ex vivo* immunization, the present invention also provides compositions and methods for *in vivo* immunization to elicit an immune response directed against an antigen in a patient.

The present invention provides a method for treating tumors comprising administering to a subject in need thereof, a therapeutically effective amount of the CAR-modified T cells of the present invention.

The CAR-modified T cells of the present invention may be administered either alone, or as a pharmaceutical composition in combination with diluents and/or with other components such as IL-2, IL-17 or other cytokines or cell populations. Briefly, pharmaceutical compositions of the present invention may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-tumor effective amount", "an tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of $10^4$ to $10^9$ cells/kg body weight, preferably $10^5$ to $10^6$ cells/kg body weight, including all integer values within those ranges. T cell compositions may also be administered multiple times at these dosages. The cells can be administered by using infusion techniques that are commonly known in immunotherapy (see, e.g., Rosenberg et al,, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (*i.v.*) injection, or intraperitoneally. In one embodiment, the T cell compositions of the present invention are administered to a patient by intradermal or subcutaneous injection. In another embodiment, the T cell compositions of the present invention are preferably administered by i.v. injection. The compositions of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or following) any number of relevant treatments, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. In further embodiments, the T cells of the present invention may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell compositions of the present invention are administered to a patient in conjunction with (e.g., before, simultaneously or following) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to practices in the field. In general, $1\times10^6$ to $1\times10^{10}$ of the modified T cells of the present invention (e.g., CAR-T20 cells) can be applied to patients by means of, for example, intravenous infusion in each treatment or each course of treatment.

**The main advantages of the present invention include:**

(a) In the present invention, CD7-CAR vectors with different structures were constructed. The *in vitro* killing effects of corresponding CAR-T cells on CD7-positive tumor cells were detected. The CD7-CAR structure with the best killing effect was selected and obtained, namely CD7-CAR (PA3-17). PA3-17 contains a humanized anti-CD7 nanobody. Compared with CARs with other structures, PA3-17 did not have the highest affinity, but surprisingly, T cells transduced with CD7-CAR had the best killing effect *in vitro*. PA3-17 is a third generation CAR, comprising an ICOS intracellular co-stimulatory region and a 4-1BB intracellular co-stimulatory region and the transmembrane region thereof is also derived from ICOS. Experiments show that said structure promotes the proliferation of T cells, which is very beneficial to transfect and amplify T cells from patients (with low activity and small number).

(b) In the present invention, CD7-blocking vectors that can block the expression of CD7 on the surface of T cells are constructed, in order to avoid the self injury of CAR-T cells targeting CD7. Compared with electroporation of the prior art, in the present invention, the expression of CD7 is blocked by lentivirus transfection. The activity of transfected T cells is better, and the blocking effect on CD7 is more stable. Moreover, the killing activity and cytokine secretion of transfected T cells as well as the phenotypes of CD4+ and CD8+ are not affected,.

(c) The CAR-T cells of the present invention have significant specific killing effect on CD7-positive malignant tumors, thereby establishing a foundation for clinically targeted therapy of T cell-related malignant tumors (T-cell leukemia and lymphoma). Surprisingly, the inventors have found that compared with normal T cells, CAR-T cells obtained by transfecting T cells from patients with CD7-CAR (PA3-17) have higher killing activity and still have better killing effect under the effector:target ratio of 1:100.

(d) CD7-CAR-T cells constructed with a humanized anti-CD7 nanobody sequence of the present invention can further reduce the *in vivo* immunogenicity of CD7-CAR-T cells constructed by camel CD7 antibody sequences and improve the clinical treatment effect.

(e) The CAR-T cells of the invention have good specific killing ability on various CD7-positive AML cell lines, and the killing effect is related to the intensity of CD7 expressed by tumor cells.

The present invention will be further explained below in conjunction with specific embodiments It should be understood that these embodiments are only used to illustrate the present invention and not to limit the scope of the present invention. For the experimental methods in the following examples, the specific conditions of which are not specifically indicated, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified. Unless otherwise specified, percentages and parts are percentages by weight and parts by weight.

**Example 1 Blocking effect of CD7-blocking molecule on CD7 molecule on surface of T cells**

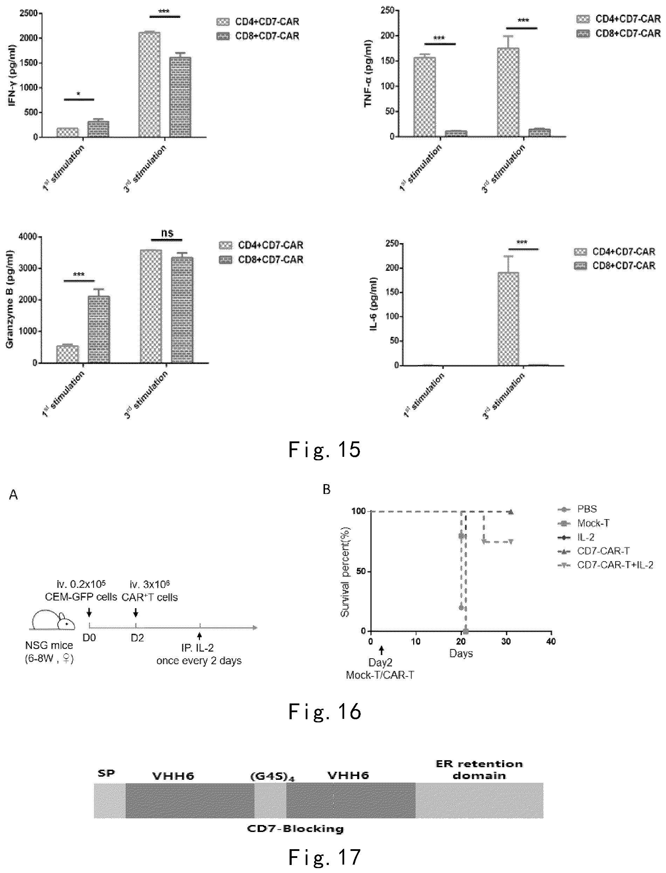

CD7-blocking molecule was constructed by using conventional technical means in the art, and the structure thereof was shown in FIG.17 and amino acid sequences was shown in SEQ ID NO: 2. The above CD7-blocking molecule was inserted into the lentiviral vectors. Then the CD7-blocking lentiviruses were prepared through lentiviral packaging technique and were used to transfect T cells. Changes of CD7 molecules on the surface of T cells were detected with flow cytometry.

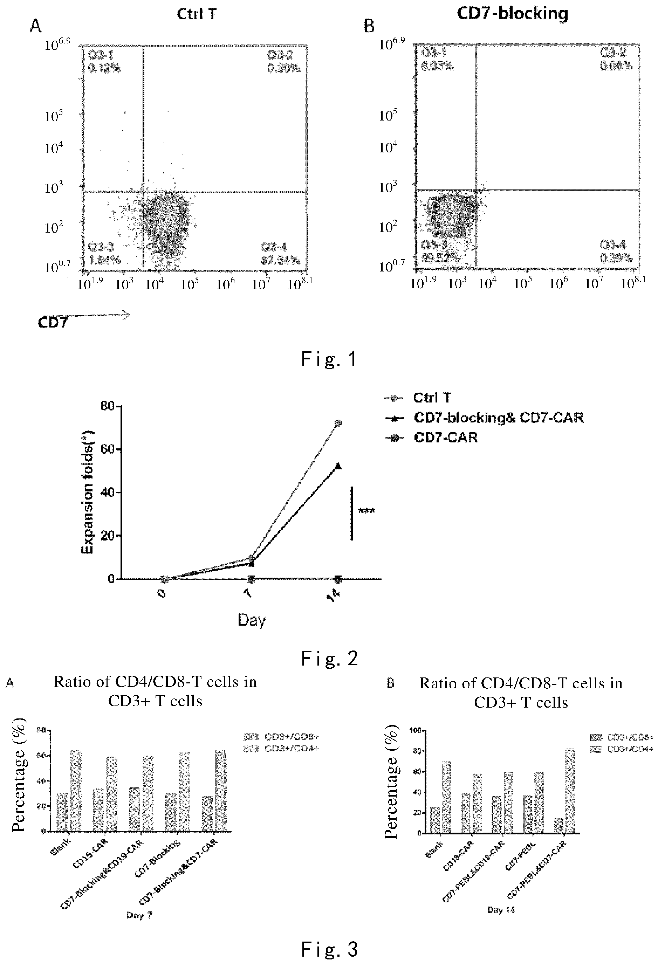

The results are shown in Fig.1. After $1\times10^6$ cells were transfected with 50ul CD7-blocking viruses, basically the expression of CD7 molecules could not be detected on the surfaces of T cells (as shown in FIG.1B), while the positive rate of CD7 on the surfaces of T cells that were not transfected with CD7-blocking viruses was 97% (as shown in FIG.1A). It is indicated that the CD7-blocking molecules constructed in this example could completely block the expression of CD7 on the surface of T cells.

**Example 2 Effect of CD7-blocking molecule on T cell expansion**

In this example, the *in vitro* expansions of control T cells not transfected with CD7-blocking virus (Ctrl T), CD7-CAR-T cells transfected with only CD7-CAR lentivirus (CD7-CAR), and CD7-CAR cells transfected with both CD7-blocking virus and CD7-CAR virus (CD7-blocking & CD7-CAR) were detected and compared.

The results are shown in Fig. 2. Compared with control T cells, CD7-CAR-T cells transfected with only CD7-CAR lentivirus (CD7-CAR) were difficult to expand *in vitro,* which was caused by the expression of CD7 molecules on the surface of CAR-T cells and the suicide of CD7-CAR-T cells. CD7-CAR-T cells transfected with both CD7-blocking virus and CD7-CAR virus (CD7-blocking & CD7-CAR) could expand normally, indicating that blocking of CD7 molecules basically did not affect the normal expansion of CD7-CAR-T cells.

**Example 3 Effect of blocking CD7 on T cell phenotype**

In this example, changes of CD4 and CD8 phenotypes on the surface of control T cells (Blank), CD19-CAR-T cells (CD19-CAR), CD7-blocked CD19-CAR-T cells (CD7-blocking & CD19-CAR), CD7 blocked T cells (CD7-blocking), CD7-blocked CD7-CAR-T cells (CD7-blocking & CD7-CAR) were analyzed.

The results of flow cytometry on Day 7 (Fig. 3A) and Day 14 (Fig.3B) showed that the blocking of CD7 molecules basically did not affect the phenotypes of CD4 cells and CD8 cells.

**Example 4 Effect of blocking CD7 on CAR-T cell function**

In order to study whether blocking CD7 molecules affected the function of CAR-T cells, CD19-CAR-T cells with blocked CD7 molecules (CD7-blocking & CD19-CAR) were constructed by using the CD7-blocking molecule in Example 1, and were detected by flow cytometry.

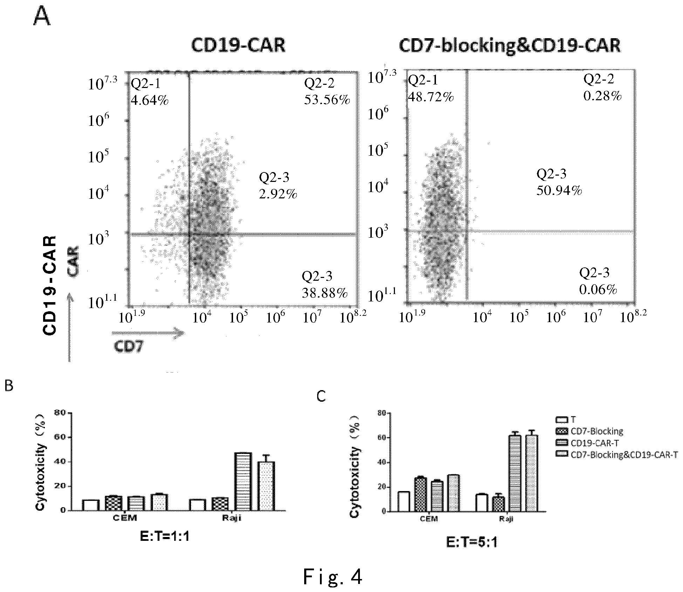

The results are shown in Fig.4A. Compared with the control CD19-CAR-T cells (CD19-CAR), the expression of CD7 molecules was basically not detected on the surface of CD7-blocked CD19-CAR-T cells (CD7-blocking & CD19-CAR).

Subsequently, the cytotoxicity of T cells, CD7-blocked T cells (CD7-blocking), CD19-CAR-T cells and CD7-blocked CD19-CAR-T cells (CD7-blocking & CD19-CAR) on Raji cells (CD19-positive tumor cells) and CEM (CD19-negative control target cells) was analyzed.

The results are shown in Fig.4. Under the condition of effector:target ratios of 1:1 and 5:1, compared with the control group (T cell group), CD7-blocked CD19-CAR-T cells (CD7-blocking&CD19-CAR) and CD7-non-blocked CD19-CAR-T cells have significant cytotoxicity on Raji cells (Fig.4B and 4C), indicating that blocking CD7 did not affect the cytotoxicity of CD19-CAR-T cells on target cells.

Then, the release of cytokines (IL-2, IFN-r, TNF-$\alpha$ and Granzyme B) by T cells, CD7-blocked T cells (CD7-blocking), CD19-CAR-T cells and CD7-blocked CD19-CAR-T cells (CD7-blocking & CD19-CAR) respectively after incubation with Raji cells was further analyzed. The effector:target ratios were 1:1 and 5:1. CEM was used as CD19-negative control target cell.

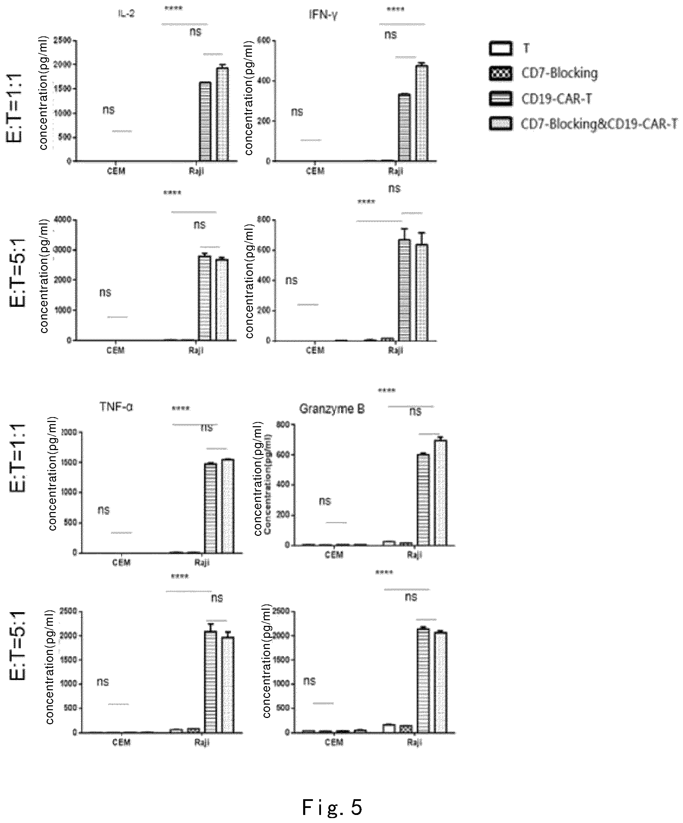

The results are shown in Fig.5. Compared with the control T cell group, CD7-blocked CD19-CAR-T cells (CD7-blocking&CD19-CAR), similar to CD7-non-blocked CD19-CAR-T cells, released significantly increased cytokines after incubation with Raji cells, further indicating that blocking CD7 did not affect the function of CAR-T cells.

**Example 5 CD7-CAR-Jurkat cells were specifically activated by target cells**

In order to verify whether five CD7-CARs (PA2-007-tEGFR, PA3-007d-tEGFR, PA3-007d-Fc, PA3-17 and PA3-17d) constructed in the present invention could be specifically activated by CD7-positive tumor cells, five CD7-CAR-Jurkat cells were constructed respectively, and then co-incubated with CEM cells respectively, and then the expression of CD69 was detected.

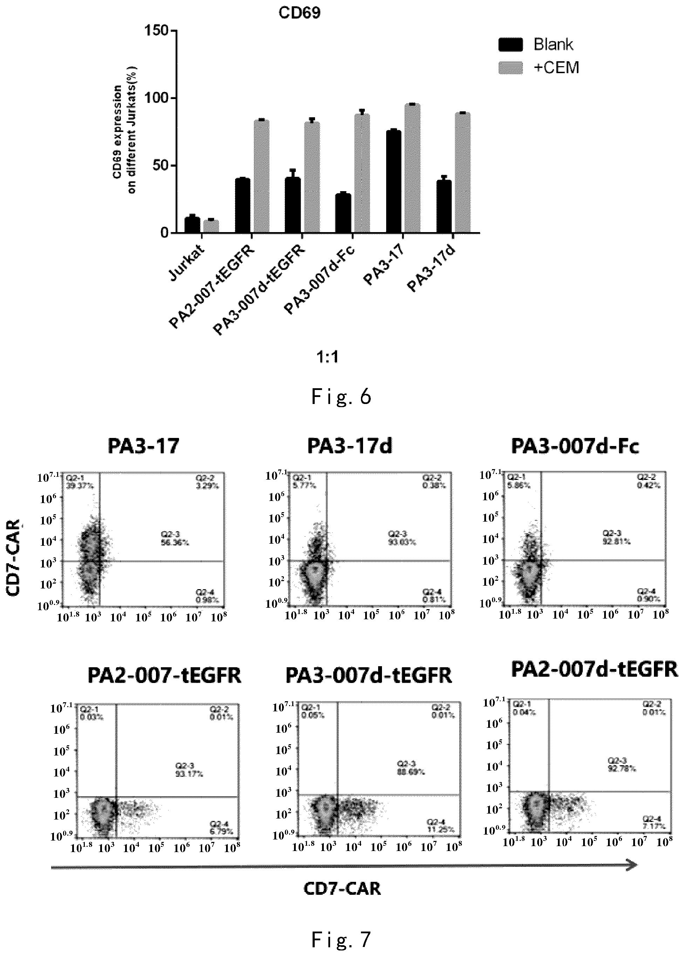

The results are shown in Fig.6. The expression of CD69 on the surface of five CD7-CAR-Jurkat cells co-incubated with CEM was significantly increased, indicating that the five CD7-CAR-Jurkat cells constructed in the present invention could be specifically activated by CD7-positive tumor cell CEM.

**Example 6 Construction and identification of CD7-CAR-T cells**

Fig. 18 shows the structure of six CD7-CARs. In order to further verify the function of each CD7-CAR, six CD7-CARF-T cells (PA3-17, Pa3-17d, PA3-007d-Fc, PA2-007-tEGFR, PA3-007-tEGFR and PA2-007-tEGFR) were constructed by lentivirus co-transfection (T cells were co-transfected with CD7-CAR lentivirus and CD7-blocking virus so as to block expression of CD7 molecules on the surface of CD7-CAR-T cells and avoid self-killing of CD7-CAR-T cells).

CAR positive rates of the CAR-T cells were detected by flow cytometry.

The results of flow cytometry are shown in Fig. 7. PA3-17 had the highest transfection efficiency.

**Example 7 CD7-CAR-T cells killed CD7-positive tumor cells *in vitro***

The *in vitro* experiments were performed on CD7-positive tumor cells CEM and Jurkat cells by using six kinds of CD7-CAR-T cells constructed in Example 6, wherein T cell group (Blank T) and CD7-blocked T cell group were used as control effector cells.

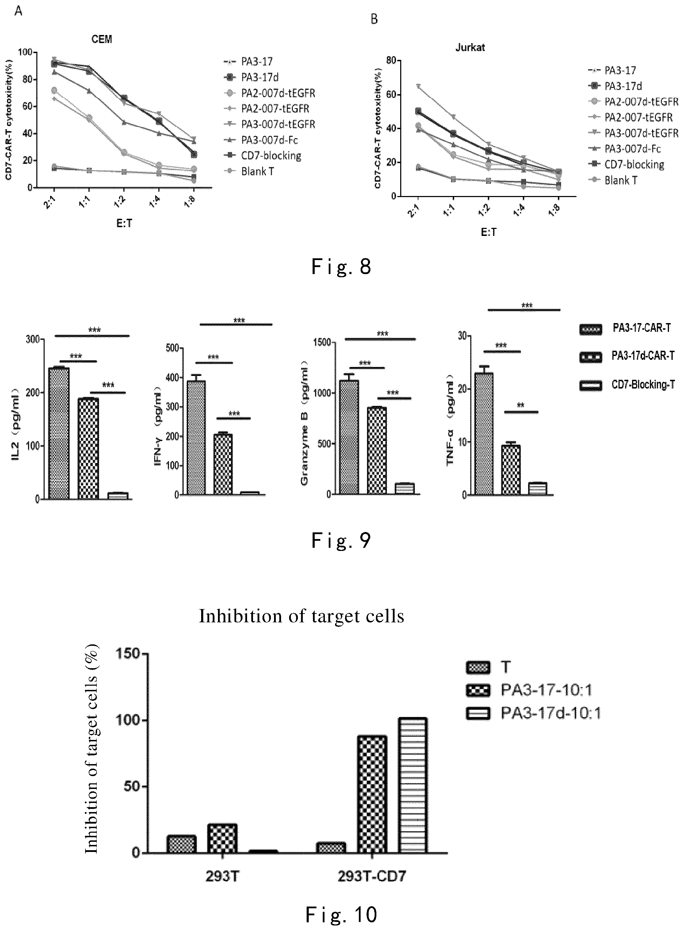

The results are shown in Fig. 8. Fig. 8A shows the cytotoxicity of six kinds of CD7-CAR-T cells on CEM. Results showed that compared with the control group, these six kinds of CD7-CAR-T cells significantly and specifically killed CEM cells under various effector:target ratios. Fig. 8B shows the cytotoxicity of six kinds of CD7-CAR-T cells on Jurkat cells. Results showed that compared with the control group, these six kinds of CD7-CAR-T cells significantly and specifically killed Jurkat cells under various effector:target ratios.

The above results showed that six kinds of CD7-CAR-T cells significantly and specifically killed CD7-positive tumor cell lines CEM and Jurkat. PA3-17-CAR-T and PA3-17d-CAR-T cells performed more prominently in *in vitro* expansion and killing experiments. Therefore, PA3-17-CAR-T and PA3-17d-CAR-T were selected and used in the following examples.

**Example 8 CD7-CAR-T cells were specifically activated by target cells**

PA3-17-CAR-T and PA3-17d-CAR-T cells were incubated with CEM cells respectively to further detect the release of cytokines (IL-2, IFN-$\gamma$, Granzyme B and TNF-$\alpha$). CD7-blocking-T was used as the negative control cell.

The results are shown in Fig. 9. Compared with CD7-blocking-T cells, two kinds of CD7-CAR-T cells (PA3-17-CAR-T and PA3-17d-CAR-T) secreted significantly increased cytokines after co-incubation with CEM cells, indicating that CD7-CAR-T cells (PA3-17-CAR-T and PA3-17d-CAR-T) could be specifically activated by CD7-positive CEM cells.

**Example 9 Inhibitory effect of CD7-CAR-T cells on CD7-overexpressing 293-T cells**

In order to further verify the specificity of CD7-CAR-T cells on killing CD7-positive target cells, a CD7-overexpressing 293T-CD7 cell line was constructed by using CD7 negative 293T cells, and the inhibitory effect of CD7-CAR-T cells (PA3-17 and PA3-17d) on the growth of 293T-CD7 cells was analyzed by RTCA instrument from ACEA Biosciences.

The results are shown in Fig. 10. Under the condition of effector:target ratio of 10:1, compared with the control T cells, CD7-CAR-T cells (PA3-17 and PA3-17d) significantly inhibited the growth of 293T-CD7 cells, while for wild-type 293T cells, CD7-CAR-T cells (PA3-17 and PA3-17d) did not show any significant specific killing activity. It proved that CD7-CAR-T cells (PA3-17 and PA3-17d) selectively killed CD7-positive target cells.

Then, the release of cytokines (IL-2, IFN-$\gamma$, Granzyme B and TNF-$\alpha$) in the above killing experiment was further analyzed.

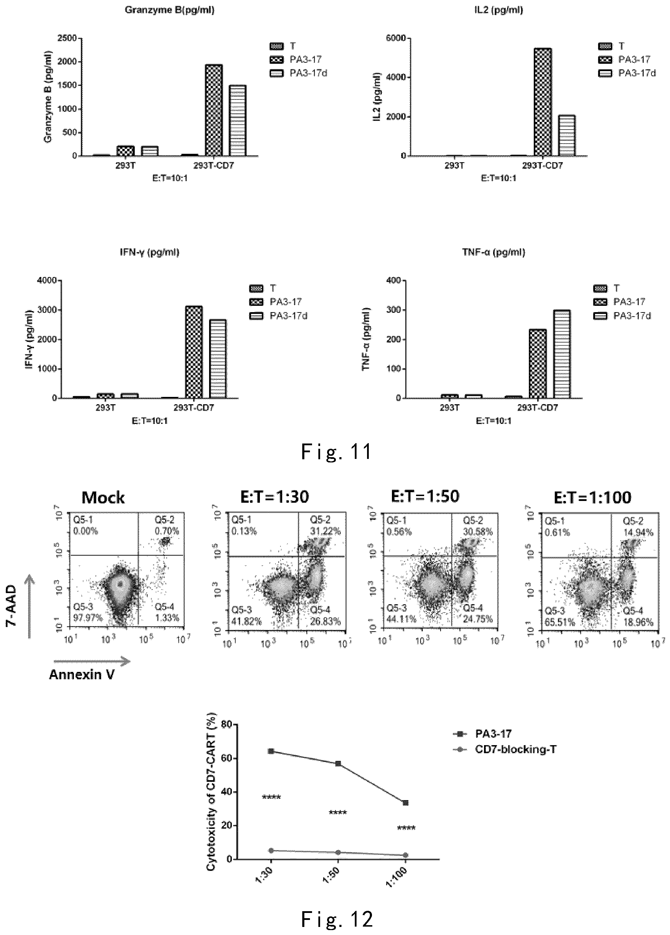

The results are shown in Fig. 11. Compared with control T cells, two kinds of CD7-CAR-T cells (PA3-17-CAR-T and PA3-17d-CAR-T) secreted significantly increased cytokines after co-incubation with CD7-overexpressing 293T-CD7 cells, while the release of cytokines by these two kinds of CD7-CAR-T cells incubated with 293T cells was almost not detected, indicating that CD7-CAR-T cells could be specifically activated by CD7-overexpressing 293T-CD7 cells, but not by CD7-negative 293T cells.

**Example 10 Cytotoxicity of CD7-CAR-T cells on CEM**

CD7-CAR-T cells (PA3-17) were prepared by using T cells from three patients. The *in vitro* killing experiments were performed on CEM tumor cells. Similar experimental results were obtained. One of the results is as follows.

The results are shown in Fig. 12. Under the condition of low effector:target ratios of 1:100, 1:50 and 1:30, compared with T cell group blocked only CD7 (CD7-blocking), CD7-CAR-T cells (PA3-17) had stronger cytotoxicity on CEM, indicating that the CD7-CAR-T cells of the present invention had significant and specific killing effect on CEM cells.

Moreover, the specific killing remained at a very low effector:target ratio (1:100), and it proved the great potential of CD7-CAR-T cells prepared by using T cells from patients as a killer for various tumors.

**Example 11 Sorting of CD4+ T cells and CD8+ T cells and preparation of CD7-CAR-T cells**

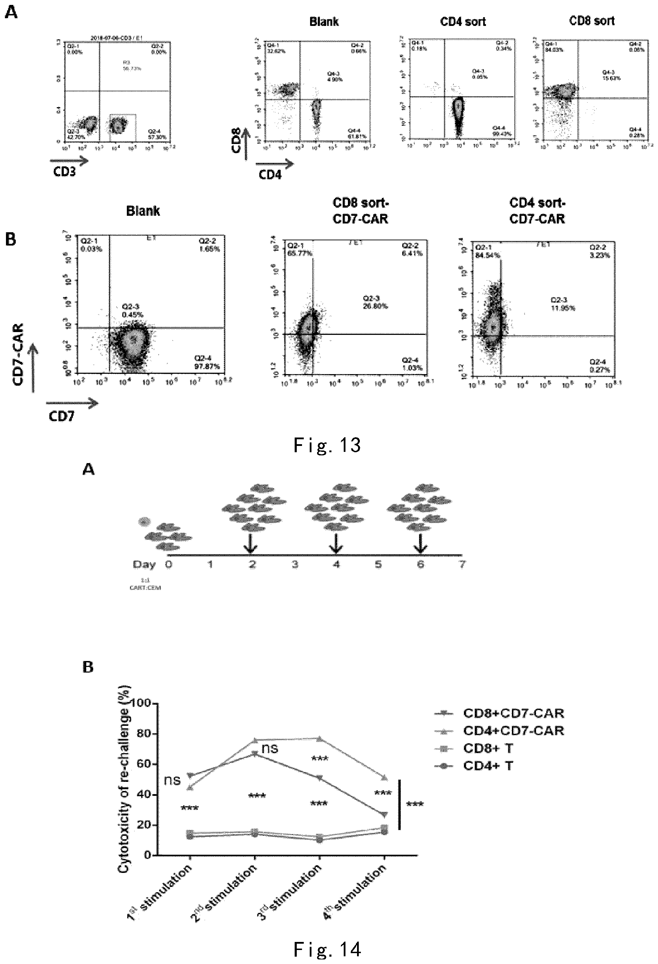

In order to further analyze the effects of CD4+ and CD8+ phenotype T cells on the biological activity of CD7-CAR-T cells, CD4+ T cells and CD8+ T cells were obtained by magnetic bead sorting. The results of flow cytometry are shown in Fig. 13A.

Then CD8+ CD7-CAR-T cells (PA3-17) and CD4+ CD7-CAR-T cells (PA3-17) were prepared respectively. The results of flow cytometry are shown in Fig. 13B.

**Example 12 CD4+ CD7-CAR-T cells and CD8+ CD7-CAR-T cells killed CEM cells *in vitro***

CD4+ CD7-CAR-T cells and CD8+ CD7-CAR-T cells prepared in Example 11 were stimulated multiple times with CEM cells, as shown in Fig. 14A. Then the cytotoxicity of CD4+ CD7-CAR-T cells and CD8+ CD7-CAR-T cells multi-stimulated by antigens on CEM cells was detected.

The results are shown in Fig. 14B. Compared with CD8+ T cells and CD4+ T cell control group, CD4+ CD7-CAR-T cells and CD8+ CD7-CAR-T cells had significant and specific killing effect on CEM. However, as the antigen stimulation times continuously increased, CD4+ CD7-CAR-T cells had more permanent killing effect on CEM cells than that of CD8+ CD7-CAR-T cells.

Then, the release of cytokines in CD4+ CD7-CAR-T cells and CD8+ CD7-CAR-T cells after the first antigen stimulation and the third antigen stimulation were further analyzed. The cytokines analyzed comprised IFN-$\gamma$, TNF-$\alpha$, Granzyme B and IL-6.

The results are shown in Fig. 15. As the antigen stimulation times increased, the cytokine release ability of CD4+ CD7-CAR-T cells was stronger than that of CD8+ CD7-CAR cells. This further indicated that after antigen multi-stimulation, CD4+ CD7-CAR-T cells might have more lasting cellular activity than that of CD8+ CD7-CAR-T cells.

**Example 13 *In vivo* antitumor activity of CD7-CAR-T cells**

In order to evaluate the *in vivo* antitumor activity of CD7-CAR-T cells, a mouse xenograft model was constructed with T-ALL cell line CEM cell. The experimental scheme of animal experiment is shown in Fig. 16A. On Day 0, each mouse was injected with $0.2 \times 10^5$ cells via tail vein. Mice were divided into 5 groups with 5 mice in each group, which were PBS group, Mock-T (T cell) group, IL-2 group, CD7-CAR-T group, CD7-CAR-T + IL2 group, respectively. Each mouse in CD7-CAR-T group was injected with $3 \times 10^6$ cells on Day 2, and each in CD7-CAR-T + IL2 group was injected with IL-2 every other day via tail vein. The survival time of mice was observed.

The results are shown in Fig. 16, compared with the control group (PBS group, Mock-T group and IL-2 group), the survival time of mice in CD7-CAR-T group and CD7-CAR-T + IL2 group was significantly prolonged (All mice in CD7-CAR-T group still currently survived, and the survival time thereof was 10 days longer than that in the control group. The experiment was not completed yet and was still under continuous observation), indicating that CD7-CAR-T cells could significantly inhibit the production of T cell tumor cells in mice and had significant antitumor effect *in vivo.*

**Example 14 Determination of antigenic epitope of anti-CD7 antibody**

In order to determine the antigen epitope recognized by the humanized anti-CD7 nanobody sequence in PA3-17-CAR, the expression vectors were prepared by using the CD7 antigen fragments, in order to express different truncated CD7 antigen fragments. The binding of anti-CD7 antibody to different truncated antigens was detected. The specific methods are as follows.

14.1 Preparation of CD7 antigen fragment expression vector

The amino acid sequence information of the extracellular segment of the target antigen CD7 was analyzed. The truncated fragments of the antigens were designed. 10 amino acids were truncated in each fragment successively. Primers were designed to perform PCR and the fragments were subcloned into the target vector Lenti-CMV-puro (IL2 signal peptide, MYRMQLLSCIALSLALVTNS (SEQ ID No:50), was used as signal peptide and EcoRI cleavage site was used).

The amino acid sequence of CD7 truncated fragments are shown in SEQ ID Nos: 33-48.

14.2 Expression and flow cytometry of different truncated forms of CD7 antigen

(a) The LVTransm transfection reagent and antibody expression vector were taken out from the refrigerator. After thawing at room temperature, they were blown with a pipette gun to be fully mixed. PBS buffer was taken out and warmed to room temperature. 0.5 ml PBS was added into each well of the 6-well plate. 6 $\mu$g different truncated CD7 expression vectors were added. The mixtures were blown with a pipette gun to be fully mixed. Then 18 $\mu$L LVTransm

was added, immediately blown with a pipette gun to be fully mixed, and left at room temperature for 10 minutes.

(b) The above DNA/LVTransm complexes were added into 1.5 ml 293F cells and shaken gently to be mixed well. The cells were placed in an incubator at 37°C and 5% $CO_2$ and incubated at 130 rpm for 6-8 hours. Then 1.5 ml fresh FreeStyle™ 293 Expression Medium was added and the cells were placed back into the incubator to continue cultivation.

(c) After continuous cultivation for 48 hours, the cells were centrifuged and collected for flow cytometry.

14.3 Detection of the binding of anti-CD7 antibody to different truncated antigens via flow cytometry

(a) The cells transfected in step 14.2 were collected and were divided into 2 parts for each type of cell, with $5 \times 10^5$ cells in each aliquot;

(B) Anti-CD7 antibodies were incubated respectively (1 ug/well), mixed well and incubated at room temperature for 1 hour;

(c) Centrifugation was performed with 800g at room temperature for 5 minutes. The supernatants containing antibody were removed. The cells were washed with PBS for 3 times;

(d) 1ul PE-labeled Anti-human IgG was added, mixed well, and incubated at room temperature in the dark for 30 min;

(e) Centrifugation was performed with 800g at room temperature for 5 minutes. The supernatants containing secondary antibody were removed. The cells were washed with PBS for 3 times;

(f) The cells were resuspended with 500ul PBS for flow cytometry.

The results of flow cytometry for the binding of anti-CD7 antibody (anti-CD7 antibody sequence in PA3-17-CAR) to different truncated antigens are shown in Fig. 19.

The results showed that the antigenic epitope of CD7 was in the first 10 amino acids of the extracellular segment. Based on the signal peptide prediction analysis, two additional amino acids would be cleaved at the cleavage position of IL2 signal peptide, so the antigenic epitope should be at amino acid position 3-12. In order to ensure the structural integrity of the antigen, further antigenic epitope detections were carried out through the scheme of point mutation. Each amino acid was mutated into Gly which had the smallest side chain. In the follow-up experiments, the point mutations at amino acid position 3-12 of the antigen were designed and each site was mutated into Gly. After gene synthesis, it was subcloned into Lenti-CMV-puro to construct the antigen mutation vectors.

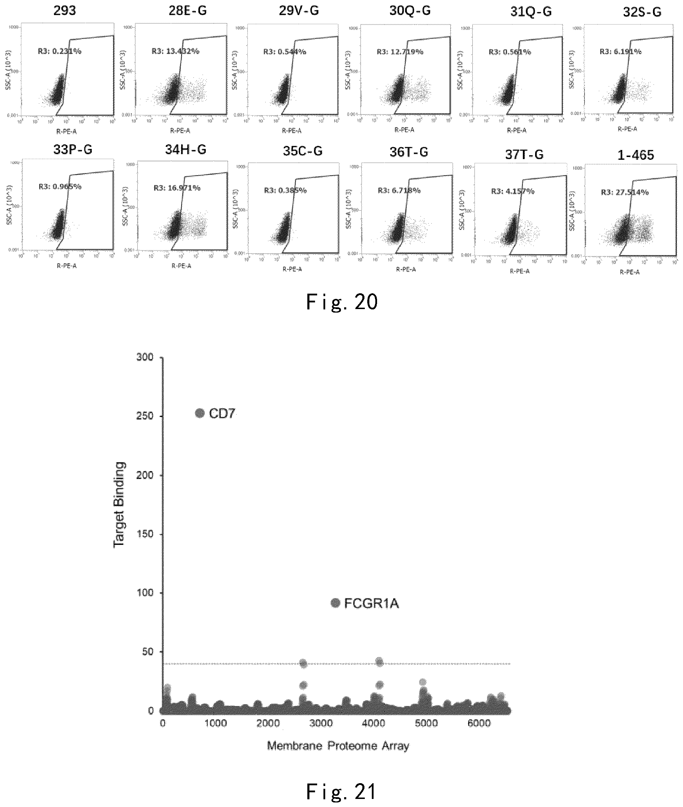

The results of flow cytometry detection of the anti-CD7 antibodies binding to antigens with mutations at different sites are shown in Fig. 20.

The results showed that the antigenic epitopes of anti-CD7 antibody were mainly in 28th- 37th amino acids, and the main sites were 29V, 31q, 33P and 35C. However, it was unable to determine by the detection results whether the specific epitope was a linear epitope or a spatial epitope.

The antigenic epitope is as follow:

MAGPPRLLLLLPLLLALARGLPGALAAQ**EVQQSPHCTT**VPVGASVNITCSTSGGLRGIYLR
QLGPQPQDIIYYEDGVVPTTDRRFRGRIDFSGSQDNLTITMHRLQLSDTGTYTCQAITEVNVYGS
GTLVLVTEEQSQGWHRCSDAPPRASALPAPPTGSALPDPQTASALPDPPAASALPAALAVISFLL
GLGLGVACVLARTQIKKLCSWRDKNSAACVVYEDMSHSRCNTLSSPNQYQ(SEQ ID NO: 49)

Among them, the underlined sequence is the signal peptide, and the bold font sequence (positions 28-37) is the detected epitope, that is, the specific antigenic epitope recognized by anti-CD7 antibody.

**Example 15 Membrane proteome array experiment of anti-CD7 antibodies**

Membrane proteome array (MPA) is a platform that can analyze the specificity of antibodies or ligands targeting human membrane proteins. MPA can be used to determine the targeting specificity of antibodies. This platform uses flow cytometry to directly detect the binding of antibodies to membrane proteins on unfixed cell surfaces. Therefore, all target proteins have their original natural conformations and appropriate post-translational modifications.

The humanized anti-CD7 nanobody sequence in PA3-17-CAR was expressed. Then the specificity and targeting property of anti-CD7 antibodies were detected by membrane proteome array to study whether they had off-target toxicity.

Specifically, the anti-CD7 antibodies of the present invention were detected with a library containing more than 5300 kinds of human membrane proteins. The library included 94% of all single transmembrane proteins, multiple transmembrane proteins and GPI targeting proteins.

The results are shown in Fig. 21. The humanized anti-CD7 nanobodies of the invention only specifically bound to CD7 protein and did not specifically bind to other membrane proteins. In addition, because the CD7 antibodies of the invention contained human Fc fragment, they would weakly bind to the human Fc receptor FCGR1A protein (not the non-specific binding of anti-CD7 antibodies), indicating that the PA3-17-CAR of the invention could only specifically recognize CD7 protein, would not bind to other proteins and would not produce off-target toxicity, thereby further proving the specificity and safety of the PA3-17-CAR of the invention.

Examples 16-19 are related to the application of CD-CAR-T cells in acute myeloid leukemia. Some materials and methods involved in the experiments are as follows.

KG-1 and Kasumi-1 cells used in the experiments were purchased from American Type Culture Center (ATCC; Manassas, VA, USA), HEL, AML5, HL-60 and THP-1 cell lines were donated by Professor Zhao Yun, Tang Zhongying hematology research center of Suzhou University. RPMI1640 and DMEM medium were purchased from Invitrogen, Tex-MACSTMGMP medium and CD3/CD28 magnetic beads were purchased from Miltenyi Biotec Co., Ltd. Fc antibodies and Human Granzyme B, Human TNF, Human IL-2 and Human IFN-$\gamma$ cytokine kits were purchased from BD company, and the apoptosis kit Annexin V-APC/7-AAD apoptosis kit was purchased from Hangzhou Lianke Biotech, Co., Ltd.

The cell culture methods involved in the experiment are as follows:

HEL cells and Kasumi-1 cells were cultured in RP-MI1640 medium supplemented with 10% FBS. KG-1 cells were cultured in IMDM medium supplemented with 10% FBS.

PBMC was extracted from normal human peripheral blood by Ficoll method and cultured in 24 well plate ($1\times10^6$ cells/well) . T cells were activated by adding 20$\mu$l CD3/CD28 magnetic beads into each well, and cultured in TexMACSTMGMP medium supplemented with IL-7 and IL-15. All cells were cultured in a cell incubator containing 5% CO$_2$, 37 °C and saturated humidity. After activated *in vitro,* the extracted T cells were transfected with CD7-CAR viruses and/or CD7-blocking viruses. The *in vitro* toxicity test was carried out when the cell proliferated to a sufficient number.

The specific killing effect of CD7-CAR T on tumor cells was detected by RTCA.

On the first day, 100 $\mu$L of 293T and 293T-CD7 tumor cells were coated on the RTCA plate in $1\times10^4$ cells/100 $\mu$L. On the second day, the effector T cells and CD7-CAR T cells were added with the effector:target ratios of 5:1 and 10:1 into the RTCA plate coated on the first day successively. The liquid medium was supplemented to 200 $\mu$l. The cell growth index was monitored every 15 mins to observe the cell growth curve. After observation, the supernatants were taken to detect cytokines.

The toxic effect of CD7-CAR T cells on tumor cells was detected by flow cytometry.

After centrifugation, $1\times10^5$ tumor cells were diluted with carboxyfluorescein successful ester (CFSE) at 1:1000, of which 100 $\mu$l were taken and incubated for 15 minutes, washed twice with PBS and coated on 24 well plates. T cells and CD7-CAR T cells were added with an effector:target ratio of 1:1, and the liquid medium was supplemented to 2 ml. After 48h, the cells were taken out from the cell incubator and co-incubation was stopped. The supernatants were retained to detect cytokines, and the apoptosis ratio of tumor cells was detected by flow cytometry after the cells were treated with apoptosis detection kit.

Detection of cytokines

The amount of cytokines produced by T cells and CD7-CAR T cells in the supernatants after co-incubation with tumor cells were detected by using the cytometric bead array (CBA) system. 50ul supernatant was taken and treated with Human Granzyme B CBA Flex Set D7 Kit, Human TNF Flex Set D9 Kit, Human IL-2 Flex Set A4 Kit and Human IFN-$\gamma$ CBA Flex Set E7 Kit. The flow cytometry was performed to detect cytokine secretion.

Statistical Analysis

Graphpad prism 5 software was used. The comparison between the two groups was tested by t-tests. **** P< 0.0001 and *P< 0.1 represent that the difference was statistically significant.

**Example 16 Screening of CD7-positive AML cell lines**

The expression of CD7 on the surface of tumor cells was detected by flow cytometry. Six AML cells, which were AML5, KG-1, HEL, HL-60, THP-1 and Kasumi-1, were collected. After centrifugation, the supernatant was removed and cells were washed with PBS (pH = 7.4). After 20 minutes of incubation with FITC-CD7 antibody (1:100), the cells were washed twice with PBS and resuspended with 500 $\mu$l PBS. The expression of CD7 on the cell surface was detected by flow cytometry.

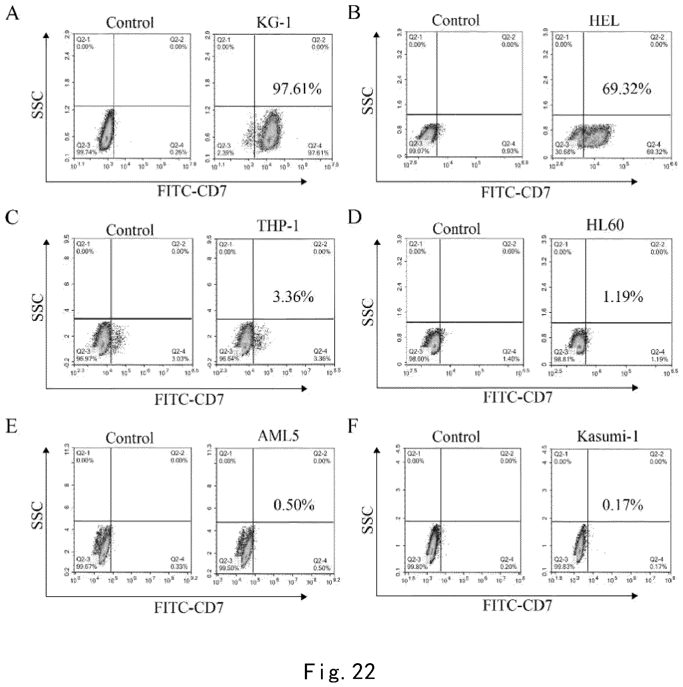

The results of flow cytometry are shown in Fig. 22. The expression rate of CD7 was 97.61% in KG-1 cells (Fig.

22a), 69.32% in HEL cells (Fig. 22b), 3.36% in THP-1 cells (Fig. 22c), 1.19% in HL-60 cells (Fig. 22d), 0.50% in AM15 cells (Fig. 22e), and 0.17% in Kasumi-1 cells (Fig.22F). Therefore, KG-1, HEL and Kasumi-1 cells (having high, medium and low expression of CD7) were selected for further experiments.

**Example 17 Constructing CD7-CAR T cells and blocking the expression of CD7 on their surface**

Due to the expression of CD7 on the surface of normal T cells, it is necessary to block the expression of CD7 on the surface of normal T cells to prevent self-killing. At present, there are two blocking methods: gene knockout and Protein Expression Blocker (PEBL), that is, the Cd7-blocking molecule constructed in Example 1, whose amino acid sequence is shown in SEQ ID No: 2. It can retain the CD7 protein on the endoplasmic reticulum and Golgi apparatus, thereby preventing the expression of the target protein. In this example, a protein blocker was used to block CD7 expression on the surface of T cells.

The Cd7-targeting CAR (CAR with co-stimulatory molecules CD28 and 41BB, whose amino acid sequence is shown in SEQ ID No: 51) was constructed by using conventional technical means in the art, which comprises anti-CD7 nanobody, human Fc sequence, co-stimulatory molecule Cd28 and 41BB sequence and CD3ζ sequence successively.

Lentiviruses were prepared according to the conventional method. T cells were activated for 48 hours and placed on 48-well plate ($5 \times 10^5$ cells/well). Virus with MOI = 30 and 15 μL Protein Expression Blocker (PEBL) were added, and the final system was 100 μl. After transfection for 15 hours, 1000 ul TexMACSTMGMP medium was added for cultivation.

The expression of CD7 on the surface of tumor cells and the transfection rate of CD7-CAR-T were detected by flow cytometry. $2 \times 10^6$ cells of CD7-CAR T cells and T cells were taken out respectively and washed once with PBS, incubated with APC-FC antibody (1:1000) for 30 minutes, washed twice with PBS, incubated with FITC-CD7 antibody (1:100) for 20 minutes, washed once with PBS, and resuspended with 500 μl PBS. The positive rate of CD7-CAR was detected by flow cytometry.

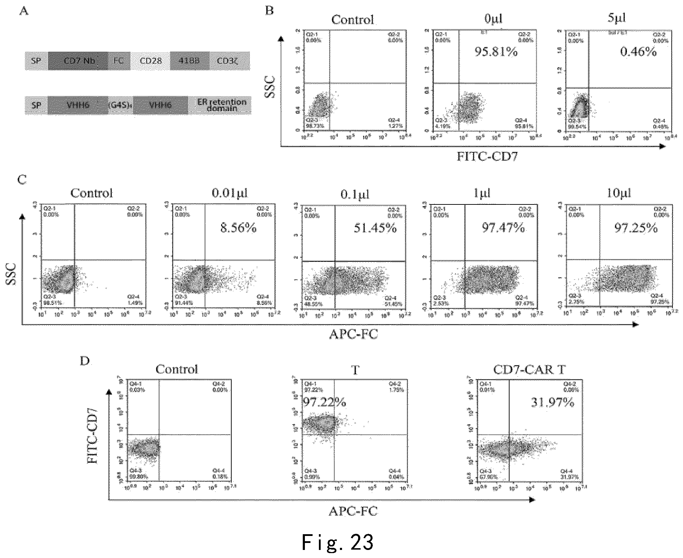

The structures of CD7-CAR and PEBL are shown in Fig. 23A, wherein CD7-CAR is a third-generation CAR composed of anti-CD7 nanobody and CD28 and 41BB co-stimulatory domains. The blocking agent was packaged into viruses and transfected into T cells. It was found that 5 μL PEBL could well block CD7 in T cells (Fig. 23B). In order to evaluate the transfection efficiency of CD7-CAR viruses, the titer was detected, and the calculated titer was about $5 \times 10^9$ (Fig.23C).

Next, after the viruses of PEBL and CD7-CAR were co-transfected into T cells, it was detected that the positive rate of CD7-CAR T cells was 31.97%, and the surface CD7 was blocked (Fig. 23D), so that the cells could be used for further cytotoxicity experiments.

**Example 18 CD7-CAR T cells specifically kill CD7-expressing tumor cells**

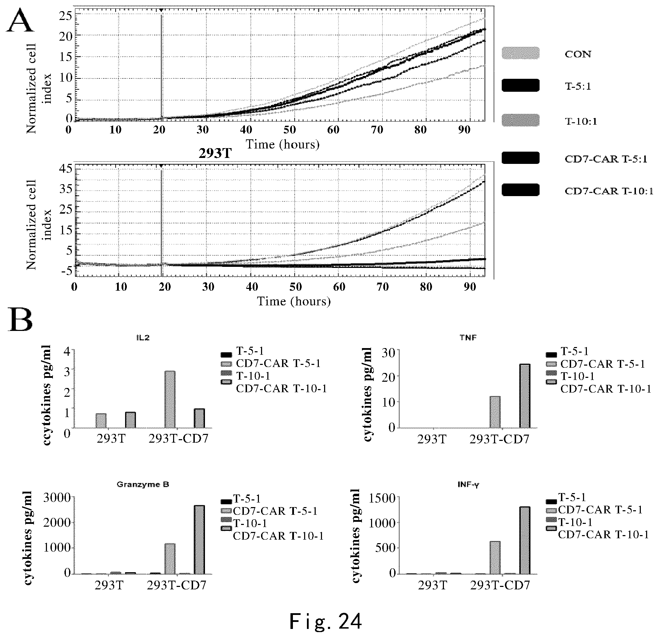

In order to verify whether CD7-CAR T could specifically kill CD7-positive tumor cells, RTCA was used to monitor the changes of tumor cell growth curve after T cells and CD7-CAR T cells were added to multi-well plate coated with 293T cells and 293T-CD7 cells, respectively. Two effector:target ratios, 5:1 and 10:1, were applied. After 96 hours, the results showed that there was no significant difference between the effect of T cells and CD7-CAR T cells on the growth of 293T cells. Compared with T cells, CD7-CAR T cells could significantly inhibit the growth of 293T-CD7 cells (Fig. 24A). The supernatant detected by RTCA was collected to detect the secretion of cytokines. The results were consistent with the growth curve. TNF, Granzyme B and INF-γ in the CD7-CAR T and 293T-CD7 cells co-incubation group were significantly higher than other groups (Fig. 24B). Therefore, the CD7-CAR T cells of the present application can specifically kill tumor cells expressing CD7 protein.

**Example 19 Specific killing ability of CD7-CAR T cells on CD7-positive cells**

T cells and CD7-CAR T cells were respectively co-incubated with KG-1 cells, HEL cells and Kasumi-1 cells for 48 hours at the effector:target ratio of 1:1. The proportions of apoptotic cells were detected by flow cytometry. The results showed that compared with T cells, almost all KG-1 cells and HEL cells co-incubated with CD7-CAR T cells were in the stage of apoptosis, while Kasumi-1 cells showed no difference between T cell group and CD7-CAR T group (E:T=1:1:t=147.1, P<0.0001; E:T=1:1:t=23.57, P<0.0001; Fig. 25A).

The cytokine secretion in co-incubated cell culture liquid was detected. Consistent with the killing results, the cytokine secretion of CD7-CAR T cell group co-incubated with tumor cells having high and moderate CD7 expression was significantly higher than that of other groups (E:T=1:1:t=4.042, P<0.5; E:T=1:1:t=52.72, P < 0.0001; E:T=1:1:t=3.787, P<0.5; E:T=1:1:t=18.59, P<0.0001; E:T=1:1:t=103.3, P<0.0001; Fig. 25B). This further proved that CD7-CAR T cells could specifically kill CD7-positive AML tumor cells.

Summary: The main contents of the present invention comprise constructing a vector that can effectively block the expression of CD7 molecule on the surface of T cells by using anti-CD7 nanobody sequence. Through a series of experiments, it showed that the CD7-blocking vector constructed in the present invention can effectively block expression of CD7 molecules on the surface of T cells. Then, six new CD7-CAR-T cells with different structures have been constructed for targeted treating CD7-positive malignant tumor cells, such as T-cell leukemia and lymphoma. The present invention discloses the structure diagrams and sequences of the sequence blocking CD7 expression on membrane and the structures and sequences of six CD7-CARs with different structures, shows the relevant *in vitro* function experimental data of different CD7-CAR-T cells on CD7-positive T cell tumor lines, CD7-overexpressing cell lines and primary T tumor cells, and shows *in vivo* antitumor effect of CD7-CAR-T cells constructed in the present invention on T cell tumor cell line CEM. To sum up, the new CD7-CAR-T cells constructed in the present invention can be used as a new treatment method for T-cell acute lymphoblastic leukemia.

All documents referred to in the present invention are incorporated by reference herein as if each document is individually incorporated by reference. Further, it should be understood that upon reading the above teaching of the present invention, various modifications or modifications may be made to the present invention by those skilled in the art, and those equivalents also fall within the scope defined by the appended claims

## Claims

1. A chimeric antigen receptor (CAR), which has an antigen binding domain that comprises one or more anti-CD7 nanobodies.

2. The CAR according to Claim 1, wherein the anti-CD7 nanobody has an amino sequence as shown in SEQ ID NO: 24.

3. The CAR according to Claim 1, wherein the CAR contains an intracellular co-stimulatory region derived from ICOS and an intracellular co-stimulatory region derived from 4-1BB.

4. A CD7-blocking molecule, which comprises one or more anti-CD7 nanobodies and an endoplasmic reticulum retention sequence.

5. A nucleic acid molecule encoding the CAR according to Claim 1 and/or the CD7-blocking molecule according to Claim 4.

6. A vector comprising the nucleic acid molecule according to Claim 5.

7. An engineered immune cell, which comprises the vector according to Claim 6 or has an exogenous nucleic acid molecule according to Claim 5 integrated into a chromosome thereof or expresses the CAR according to Claim 1 and/or the CD7-blocking molecule according to Claim 4.

8. A use of the CAR according to Claim 1, the CD7-blocking molecule according to Claim 4, or the engineered immune cell according to Claim 7 in preparation of a medicament or a formulation for preventing and/or treating tumor or cancer.

9. A method for preparing an engineered immune cell, which comprises the following steps:

(1) providing an immune cell to be modified;
(2) transducing a first expression cassette expressing the CAR according to Claim 1 into the immune cell; and
(3) transducing a second expression cassette expressing the CD7-blocking molecule according to Claim 4 into the immune cell, thereby obtaining the engineered immune cell.

10. An reagent combination, which comprises:

(1) a first virus vector, which comprises a first expression cassette expressing the CAR according to Claim 1; and
(2) a second virus vector, which comprises a second expression cassette expressing the CD7-blocking molecule according to Claim 4.

F i g. 1

F i g. 2

F i g. 3

A

B

C

Fig. 4

Fig. 5

Fig. 6

Fig. 7

Fig. 8

Fig. 9

Inhibition of target cells

Fig. 10

Fig. 11

Fig. 12

**A**

**B**

Fig. 13

**A**

**B**

Fig. 14

Fig. 15

Fig. 16

Fig. 17

F i g. 18

F i g. 19

Fig. 20

Fig. 21

Fig. 22

Fig. 23

Fig. 24

Fig. 25

## INTERNATIONAL SEARCH REPORT

| International application No. |
| --- |
| **PCT/CN2020/130945** |

**A.   CLASSIFICATION OF SUBJECT MATTER**

C07K 16/18(2006.01)i;  A61K 38/05(2006.01)i;  A61P 37/02(2006.01)i;  C12N 5/10(2006.01)i;  C12N 15/62(2006.01)i

According to International Patent Classification (IPC) or to both national classification and IPC

**B.   FIELDS SEARCHED**

Minimum documentation searched (classification system followed by classification symbols)

C07K,A61K,A61P,C12N

Documentation searched other than minimum documentation to the extent that such documents are included in the fields searched

Electronic data base consulted during the international search (name of data base and, where practicable, search terms used)

CNABS, SIPOABS, CNTXT, EPTXT, USTXT, WOTXT, DWPI, CNKI, PubMed, ISI web of knowledge, google scholar: 嵌合抗原受体, 抗体, 纳米, CAR, CD7纳米抗体, 内源性, 抗原结合结构域, CAR-T细胞, CD7-CAR-T cell, chimeric antigen receptor (CAR), antigen binding domain, CD7 nanobodies, endogenous CD7 expression

**C.   DOCUMENTS CONSIDERED TO BE RELEVANT**

| Category* | Citation of document, with indication, where appropriate, of the relevant passages | Relevant to claim No. |
| --- | --- | --- |
| X | CN 109652379 A (PERSONGEN BIOMEDICINE (SUZHOU) CO., LTD.) 19 April 2019 (2019-04-19)<br>claims 1-10, sequence 4, description, paragraphs 0097-0184 | 1-10 |
| A | CN 108884164 A (CELL MEDICA SWITZERLAND AG. et al.) 23 November 2018 (2018-11-23)<br>entire document | 1-10 |
| A | CN 109912683 A (HANGZHOU DAC BIOTECH CO., LTD.) 21 June 2019 (2019-06-21)<br>entire document | 1-10 |
| A | CN 109306341 A (EAST CHINA NORMAL UNIVERSITY et al.) 05 February 2019 (2019-02-05)<br>entire document | 1-10 |
| A | CN 106795217 A (BLUEBIRD BIO, INC.) 31 May 2017 (2017-05-31)<br>entire document | 1-10 |
| A | WO 2016154055 A1 (BLUEBIRD BIO, INC.) 29 September 2016 (2016-09-29)<br>entire document | 1-10 |

☑ Further documents are listed in the continuation of Box C.     ☑ See patent family annex.

| * Special categories of cited documents: | "T" later document published after the international filing date or priority date and not in conflict with the application but cited to understand the principle or theory underlying the invention |
| --- | --- |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **26 January 2021** | **24 February 2021** |

| Name and mailing address of the ISA/CN | Authorized officer |
| --- | --- |
| **China National Intellectual Property Administration (ISA/ CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2020/130945** |

**C.     DOCUMENTS CONSIDERED TO BE RELEVANT**

| Category* | Citation of document, with indication, where appropriate, of the relevant passages | Relevant to claim No. |
| --- | --- | --- |
| A | WO 2016187216 A1 (BLUEBIRD BIO, INC. et al.) 24 November 2016 (2016-11-24)<br>      entire document | 1-10 |
| PX | CN 110760007 A (PERSONGEN BIOMEDICINE (SUZHOU) CO., LTD.) 07 February 2020<br>(2020-02-07)<br>      claims 1-10, and abstract | 1-10 |

Form PCT/ISA/210 (second sheet) (January 2015)

## INTERNATIONAL SEARCH REPORT

| International application No. |
| --- |
| **PCT/CN2020/130945** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

           ☑ in the form of an Annex C/ST.25 text file.

           ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

           ☐ in the form of an Annex C/ST.25 text file (Rule 13*ter*.1(a)).

           ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

**INTERNATIONAL SEARCH REPORT**
Information on patent family members

International application No.

**PCT/CN2020/130945**

| Patent document cited in search report | | | Publication date (day/month/year) | Patent family member(s) | | | Publication date (day/month/year) |
|---|---|---|---|---|---|---|---|
| CN | 109652379 | A | 19 April 2019 | WO | 2020135870 | A1 | 02 July 2020 |
| CN | 108884164 | A | 23 November 2018 | AU | 2017223846 | A1 | 23 August 2018 |
| | | | | US | 2019048085 | A1 | 14 February 2019 |
| | | | | KR | 20180111870 | A | 11 October 2018 |
| | | | | WO | 2017144681 | A1 | 31 August 2017 |
| | | | | CA | 3014001 | A1 | 31 August 2017 |
| | | | | CA | 3014067 | A1 | 31 August 2017 |
| | | | | SG | 11201807188V | A | 27 September 2018 |
| | | | | BR | 112018067696 | A2 | 08 January 2019 |
| | | | | US | 2019055312 | A1 | 21 February 2019 |
| | | | | EP | 3420001 | A1 | 02 January 2019 |
| | | | | SG | 11201807187 X | A | 27 September 2018 |
| | | | | WO | 2017147383 | A8 | 15 November 2018 |
| | | | | EP | 3420000 | A1 | 02 January 2019 |
| | | | | BR | 112018067698 | A2 | 08 January 2019 |
| | | | | CN | 108699153 | A | 23 October 2018 |
| | | | | JP | 2019513009 | A | 23 May 2019 |
| | | | | KR | 20190003938 | A | 10 January 2019 |
| | | | | RU | 2018128462 | A | 25 March 2020 |
| | | | | AU | 2017224843 | A1 | 23 August 2018 |
| | | | | WO | 2017147383 | A1 | 31 August 2017 |
| | | | | JP | 2019515646 | A | 13 June 2019 |
| | | | | RU | 2018128464 | A | 25 March 2020 |
| CN | 109912683 | A | 21 June 2019 | None | | | |
| CN | 109306341 | A | 05 February 2019 | None | | | |
| CN | 106795217 | A | 31 May 2017 | AU | 2015292590 | A1 | 16 February 2017 |
| | | | | RU | 2017106025 | A | 30 August 2018 |
| | | | | US | 2017226216 | A1 | 10 August 2017 |
| | | | | KR | 20170036021 | A | 31 March 2017 |
| | | | | AU | 2015292590 | B2 | 16 January 2020 |
| | | | | EP | 3172231 | A4 | 10 January 2018 |
| | | | | JP | 6706244 | B2 | 03 June 2020 |
| | | | | BR | 112017001498 | A2 | 20 February 2018 |
| | | | | JP | 2017522882 | A | 17 August 2017 |
| | | | | IL | 250202 | D0 | 30 March 2017 |
| | | | | WO | 2016014789 | A3 | 17 March 2016 |
| | | | | WO | 2016014789 | A2 | 28 January 2016 |
| | | | | EP | 3172231 | A2 | 31 May 2017 |
| | | | | CA | 2956002 | A1 | 28 January 2016 |
| | | | | SG | 11201700492 S | A | 27 February 2017 |
| | | | | SG | 10201900455 Y | A | 27 February 2019 |
| | | | | MX | 2017001079 | A | 12 September 2017 |
| | | | | IL | 250202 | A | 30 September 2020 |
| | | | | US | 2020079864 | A1 | 12 March 2020 |
| | | | | RU | 2017106025 | A3 | 15 February 2019 |
| WO | 2016154055 | A1 | 29 September 2016 | EP | 3270960 | A1 | 24 January 2018 |
| | | | | CN | 107614008 | A | 19 January 2018 |
| | | | | JP | 2018510160 | A | 12 April 2018 |
| | | | | US | 2018094280 | A1 | 05 April 2018 |

Form PCT/ISA/210 (patent family annex) (January 2015)

**INTERNATIONAL SEARCH REPORT**
Information on patent family members

International application No.

**PCT/CN2020/130945**

| Patent document cited in search report | | | Publication date (day/month/year) | Patent family member(s) | | | Publication date (day/month/year) |
|---|---|---|---|---|---|---|---|
| | | | | AU | 2016235421 | A1 | 12 October 2017 |
| | | | | EP | 3270960 | A4 | 08 August 2018 |
| WO | 2016187216 | A1 | 24 November 2016 | CN | 108137669 | A | 08 June 2018 |
| | | | | EP | 3298032 | A1 | 28 March 2018 |
| | | | | JP | 2018521004 | A | 02 August 2018 |
| | | | | US | 2018147271 | A1 | 31 May 2018 |
| CN | 110760007 | A | 07 February 2020 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5399346 A
- US 5580859 A
- US 5589466 A
- WO 0196584 A
- WO 0129058 A
- US 6326193 B
- US 5350674 A
- US 5585362 A

**Non-patent literature cited in the description**

- **UI-TEI et al.** *FEBS Letters,* 2000, vol. 479, 79-82
- **SAMBROOK et al.** Molecular Cloning: A Laboratory Manual. Cold Spring Harbor Laboratory, 2001
- **ROSENBERG et al.** *New Eng. J. of Med.,* 1988, vol. 319, 1676
- **SAMBROOK et al.** Molecule Clone: A Laboratory Manual. Cold Spring Harbor Laboratory Press, 1989